# EUROPEAN PATENT SPECIFICATION

(11) **EP 0 858 505 B1**
(45) Date of publication and mention of the grant of the patent: **29.12.2004**
(21) Application number: 96936622.8
(22) Date of filing: 18.10.1996
(51) Int. Cl.: C12N 15/00

(54) **CLONING OF VERTEBRATE PHEROMONE RECEPTORS AND USES THEREOF**
KLONIERUNG VON PHEROMON REZEPTOREN AUS VERTEBRATEN UND DEREN VERWENDUNGEN
CLONAGE DE RECEPTEURS DE PHEROMONES DE VERTEBRES ET APPLICATIONS

(30) Priority: 19.10.1995 US 5698 P
(43) Date of publication of application: 19.08.1998
(73) Proprietor: THE TRUSTEES OF COLUMBIA UNIVERSITY IN THE CITY OF NEW YORK, New York, New York 10027 (US)
(72) Inventor: DULAC, Catherine, Cambridge, MA 02138 (US); AXEL, Richard, New York, NY 10025 (US)
(74) Representative: VOSSIUS & PARTNER
(86) International application number: PCT/US1996/016637
(87) International publication number: WO 1997/014790

(56) References cited:
- WO-A-94/28904
- US-A- 5 514 545
- MONTI-BLOCH L ET AL: "THE HUMAN VOMERONASAL SYSTEM" PSYCHONEUROENDOCRINOLOGY, OXFORD, GB, vol. 19, no. 5/7, 1994, pages 673-686, XP000863096 ISSN: 0306-4530
- ZHANG L ET AL: "Whole genome amplification from single cell: Implications for genetic analysis" PROCEEDINGS OF THE NATIONAL ACADEMY OF SCIENCES OF USA, NATIONAL ACADEMY OF SCIENCE. WASHINGTON, US, vol. 89, July 1992 (1992-07), pages 5847-5851, XP002130909 ISSN: 0027-8424
- BRADY G ET AL: "REPRESENTATIVE IN-VITRO COMPLEMENTARY DNA AMPLIFICATION FROM INDIVIDUAL HEMOPOIETIC CELLS AND COLONIES" METHODS IN MOLECULAR AND CELLULAR BIOLOGY, vol. 2, no. 1, 1990, pages 17-25, XP001013409 ISSN: 0898-7750
- WATSON JOSEPH B ET AL: "Differential cDNA screening strategies to identify novel stage-specific proteins in the developing mammalian brain." DEVELOPMENTAL NEUROSCIENCE, vol. 15, no. 2, 1993, pages 77-86, XP001012772 ISSN: 0378-5866
- CELL, Volume 83, issued 20 October 1995, TROEMEL et al., "Divergent seven Transmembrane Receptors are Candidate Chemosensory Receptors in C. Elegans", pages 207-218, XP001008859
- PROCEEDNGS OF THE NATIONAL ACADEMY OF SCIENCES, Volume 84, issued March 1987, ROGERS et al., "Molecular Cloning and Sequencing of a cDNA for Olfactory Marker Protein", pages 1704-1708, XP002905055
- CELL, Volume 79, issued 16 December 1994, VASSAR et al., "Topographic Organization of Sensory Projections to the Olfactory Bulb", pages 981-991, XP002905056
- CELL, Volume 65, issued 05 April 1991, BUCK et al., "A Novel Multigene Family May Encode Odorant Receptors: A Molecular Basis for Odor Recognition", Pages 175-187, XP002029935
- CELL, Volume 83, issued 20 October 1995, DULAC et al., "A Novel Family of Genes Encoding Putative Pheromone Receptors in Mammals", pages 195-206, XP002156690
- THE EMBO JOURNAL, Volume 12, No. 4, issued 1993, BALDWIN et al., "The Probable Arrangement of the Helices in G Protein-Coupled Receptors", pages 1693-1703, XP002905057
- NEURON, Volume 13, issued October 1994, SHEPHERD G., "Discrimination of Molecular Signals by the Olfactory Receptor Neuron", pages 771-790, XP002905073
- ADVANCES IN GENETICS, Volume 24, issued 1987, SCANGOS et al., "Gene Transfer Into Mice", pages 285-321, XP002905074

## Description

This application claims the benefit of U.S. Provisional Application No. 60/005,698, filed October 19, 1995.

The invention disclosed herein was made with Government support under NIH Grant No. NS 29832-04 from the Department of Health and Human Services. Accordingly, the U.S. Government has certain rights in this invention.

### Background of the Invention

Throughout this application, various references are referred to by abbreviation.

Full bibliographic citation for these references may be found at the end of this application, preceding the claims.

In mammals, olfactory sensory perception is mediated by two anatomically and functionally distinct sensory organs: the main olfactory epithelium (MOE) and the vomeronasal organ (VNO) Pheromones activate the VNO and elicit a characteristic array of innate reproductive and social behaviors, along with dramatic neuroendocrine responses. Differential screening of cDNA libraries constructed from single sensory neurons from the rat VNO has led to the isolation of a family of about 30 putative receptor genes. Sequence analysis indicates that these genes comprise a novel family of seven transmembrane domain proteins unrelated to the receptors expressed in the MOE. Moreover, the expression of each member of the gene family is restricted to a small subpopulation of VNO neurons. These genes encode mammalian pheromone receptors.

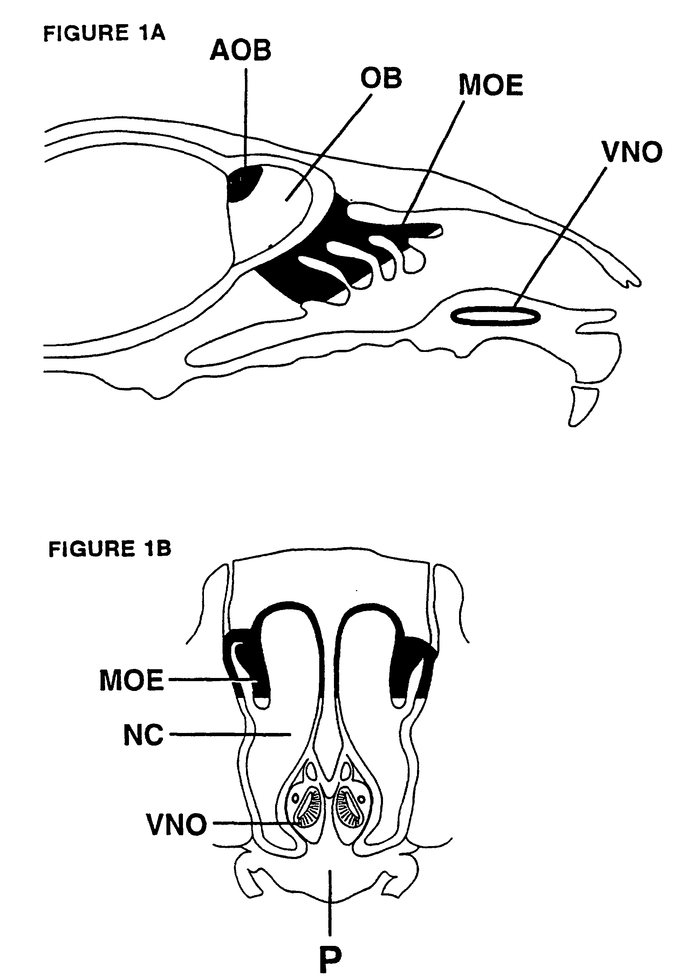

Sensory systems receive information from the environment and transmit these signals to higher cortical centers in the brain where they are processed to provide an internal representation of the external world. Mammals possess an olfactory system of enormous discriminatory power. Humans, for example, are capable of recognizing thousands of discrete odors. The perception of odors in humans is often viewed as an aesthetic sense, a sense capable of evoking emotion and memory leading to measured thoughts and behaviors. Smell, however, is also the primal sense. In most species, odors can elicit innate and stereotyped behaviors that are likely to result from the nonconscious perception of odors. These different pathways of olfactory sensory processing are thought to be mediated by two anatomically and functionally distinct olfactory sensory organs, the main olfactory epithelium (MOE) and the vomeronasal organ (VNO) (Figure 1).

In mammals, the sensory epithelium of the main olfactory system resides within the posterior recess of the nasal cavity, whereas the vomeronasal organ resides more anteriorly in a blind-ended pouch within the septum of the nose (Jacobson, 1811; see also Halpern, 1987; Wysocki, 1989; and Farbman, 1992 for reviews). The sensory neurons of both the MOE and VNO are bipolar. The dendrites terminate in specialized microvilli or cilia that bind odorants and transduce specific odorant binding into neural activity. The axons from sensory neurons of the MOE project through the skull to the main olfactory bulb, the first relay station in the brain. The main olfactory bulb then sends most of its fibers to the olfactory cortex which in turn projects to higher sensory centers. The vomeronasal system, however, transmits olfactory information via a separate pathway of neuronal projections. The neurons of the VNO send axons to the accessory olfactory bulb which projects to a discrete locus within the amygdala distinct from the zone that receives fibers from the main olfactory pathway (Broadwell, 1975; Scalia and Winans, 1975; Winans and Scalia, 1970). The vomeronasal nucleus in the amygdala, in turn, sends fibers directly to the hypothalamus (Kevetter and Winans, 1981; Krettek and Price, 1977; 1978). Thus, the VNO pathway bypasses higher cognitive centers resulting in innate and stereotyped behavioral and neuroendocrine responses.

What chemical signals activate the VNO and what responses do they elicit? The VNO is largely responsive to olfactory cues secreted by other individuals within a species. These chemical signals provide information about gender, dominance, or reproductive status and elicit innate social and sexual behaviors, along with profound neuroendocrine changes (reviewed in Halpern, 1987; Wysocki, 1989; Wysocki and Lepri, 1991). In male rodents, for example, removal of the vomeronasal organ in virgin animals severely impairs sexual responses resulting in a dramatic reduction in the frequency of mating (Clancy et al., 1984; Meredith, 1986). In female rodents, activation of the VNO can induce puberty and estrus in the presence of males and prevent estrus in group-housed females (Lomas, 1982; Johns et al., 1978; Reynolds and Keverne, 1979). Similarly, lesions in the vomeronasal system dramatically diminish male-specific aggressive behaviors (Bean, 1982; Clancy et al., 1984).

The chemical-signals responsible for eliciting these behaviors have been broadly defined as pheromones. Two classes of steroids, 16-androstenes and estrogens can elicit reproductive behaviors in some mammals (Melrose et al., 1971; Michael and Keverne, 1968); F-prostaglandins and steroids elicit sperm production and mating in fish (Stacey and Sorensen, 1986; Sorensen et al., 1988) and small fatty acids in association with the protein, aphrodisin, have been implicated in the male sexual response in hamsters (Henzel, et al., 1988; Singer, 1991). In most instances however, the chemical nature of the odorants responsible for innate behavioral responses has not been elucidated.

Neither the pheromone receptors nor the signal transduction pathways activated by pheromone in vomeronasal neurons have been identified. In the MOE, the repertoire of odorant receptor genes consists of about 1,000 genes, each encoding a distinct seven transmembrane domain protein (Buck and Axel, 1991; Parmentier et al., 1992; Ben Arie et al., 1994). Analysis of the expression patterns of this family of odorant receptor genes (Ngai et al., 1993; Ressler et al., 1993; Vassar et al., 1993; Vassar et al., 1994; Ressler et al. 1994) , coupled with earlier electrophysiologic and tracing experiments (Kauer et al., 1987; Stewart et al., 1979; Lancet et al., 1982; Mori et al., 1992; Imamura et al., 1992; Katoh et al., 1993) have provided a logic for olfactory discrimination. Individual sensory neurons in the MOE are likely to express only one of the thousand receptor genes (Ngai et al., 1993; Chess et al., 1994; C. Dulac and R. Axel, unpublished studies). Neurons expressing a given receptor, although randomly distributed in domains of the epithelium, project their axons to a small number of topographically fixed loci (or glomeruli) in the main olfactory bulb (Vassar et al, 1994; Ressler et al., 1994). These data support a model of olfactory coding in which discrimination of odor quality would result from the detection of specific spatial patterns of activity in the olfactory bulb.

The isolation of the genes encoding the pheromone receptors from VNO neurons might similarly provide insight into the chemical nature of the pheromones themselves, the logic of olfactory coding in the VNO, and the way in which perception of this class of odors leads to innate behaviors. Applicants' efforts to identify the genes encoding the mammalian pheromone receptors by virtue of potential homology with the family of odorant receptor genes expressed in the main olfactory epithelium have been unsuccessful. Applicants therefore developed a cloning strategy in which cDNA libraries were constructed from individual rat VNO neurons. Difference cloning permitted the identification of about 30 genes that define a novel family of presumed seven-transmembrane domain receptors that are evolutionarily independent of the odorant receptors of the MOE. Expression of the individual members of this gene family is restricted to a distinct set of VNO neurons such that different neurons express different receptor genes. These genes encode mammalian pheromone receptors.

### Summary of the Invention

This invention provides an isolated nucleic acid molecule encoding a vertebrate pheromone receptor. This invention also provides a nucleic acid molecule of at least 12 nucleotides capable of specifically hybridizing with a unique sequence within the sequence of the above-described nucleic acid molecule.

This invention provides a vector which comprises the above-described isolated nucleic acid molecule. In an embodiment, the above described isolated nucleic acid molecule is operatively linked to a regulatory element. In a further embodiment, the vector is a plasmid.

This invention provides a host vector system for the production of a polypeptide having the biological activity of a vertebrate pheromone receptor which comprises the above-described vector and a suitable host. This invention also provides a host vector system, wherein the suitable host is a bacterial cell, yeast cell, insect cell, or animal cell.

This invention provides a method of producing a polypeptide having the biological activity of a vertebrate pheromone receptor which comprising growing the above-described host vector system under conditions permitting production of the polypeptide and recovering the polypeptide so produced.

This invention also provides a purified, vertebrate pheromone receptor.

This invention also provides a polypeptide encoded by the above-described isolated vertebrate nucleic acid molecule.

This invention provides an antibody capable of binding to a vertebrate pheromone receptor. This invention further provides an antibody capable of competitively inhibiting the binding of the antibody capable of binding to a vertebrate pheromone receptor.

This invention provides a method for identifying cDNA inserts encoding pheromone receptors comprising: (a) generating a cDNA library which contains clones carrying cDNA inserts from an individual vomeronasal sensory neuron;(b) hybridizing the nucleic acid molecules of clones from the cDNA libraries generated in step (a) with probes prepared from the individual vomeronasal neuron and probes from a second individual vomeronasal neuron or a main olfactory epithelium neuron; (c) selecting clones which hybridized with probes from the individual vomeronasal neuron but not from the second individual vomeronasal neuron or the main olfactory epithelium neuron; and (d) isolating clones which carry the hybridized inserts, thereby identifying the inserts encoding pheromone receptors.

This invention also provides the above-described method wherein after step (c), further comprising: (a) amplifying the inserts from the selected clones by polymerase chain reaction; (b) hybridizing the amplified inserts with probes from the individual vomeronasal neuron; and (c) isolating the clones which carry the hybridized inserts, thereby identifying the inserts encoding the pheromone receptors. This invention also provides cDNA inserts identified by the above methods.

This invention also provides a method for identifying DNA inserts encoding pheromone receptors comprising: (a) generating DNA libraries which contain clones carrying inserts from a sample containing vomeronasal sensory neuron(s); (b) contacting clones from the cDNA libraries generated in step (a) with nucleic acid molecule of at least 12 nucleotides capable of specifically hybridizing with a unique sequence within the sequence of a pheromone receptor in appropriate conditions permitting the hybridization of the nucleic acid molecules of the clones and the nucleic acid molecule; (c) selecting clones which hybridized with nucleic acid molecule; and (d) isolating the clones which carry the hybridized inserts, thereby identifying the inserts encoding the pheromone receptors. In an embodiment, the sample only contain an individual vomeronasal sensory neuron.

This invention also provides a method to identify DNA inserts encoding pheromone receptors comprising: (a) generating DNA libraries which contain clones with inserts from a sample containing vomeronasal sensory neuron(s); (b) contacting the clones from the DNA libraries generated in step (a) with appropriate polymerase chain reaction primers capable of specifically binding to nucleic acid molecules encoding pheromone receptors in appropriate conditions permitting the amplification of the hybridized inserts by polymerase chain reaction; (c) selecting the amplified inserts; and (d) isolating the amplified inserts, thereby identifying the inserts encoding the pheromone receptors.

This invention also provides DNA inserts identified by the above methods.

This invention provides a method to isolate DNA molecules encoding pheromone receptors comprising: (a) contacting a biological-sample known to contain nucleic acids with appropriate polymerase chain reaction primers capable of specifically binding to nucleic acid molecules encoding pheromone receptors in appropriate conditions permitting the amplification of the hybridized molecules by polymerase chain reaction; (b) isolating the amplified molecules, thereby identifying the DNA molecules encoding the pheromone receptors. This invention also provides the nucleic acid molecules isolated by the above method.

This invention provides a method of transforming cells which comprises transfecting a host cell with a suitable vector comprising a nucleic acid molecule encoding a pheromone receptor as described above. This invention also provides transformed cells produced by above method.

The invention also provides transformed cells wherein the host cells are not usually expressing pheromone receptors and transformed cells wherein the host cells are expressing pheromone receptors.

This invention provides a method of identifying a compound capable of specifically binding to a vertebrate pheromone receptor which comprises contacting a transfected cells or membrane fractions of the above transfected cells with an appropriate amount of the compound under conditions permitting binding of the compound to such receptor, detecting the presence of any such compound specifically bound to the receptor, and thereby determining whether the compound specifically binds to the receptor.

This invention provides a method of identifying a compound capable of specifically binding to a vertebrate pheromone receptor which comprises contacting an appropriate amount of the purified pheromone receptor with an appropriate amount of the compound under conditions permitting binding of the compound to such purified receptor, detecting the presence of any such compound specifically bound to the receptor, and thereby determining whether the compound specifically binds to the receptor.

This invention also provides a method of identifying a compound capable of activating the activity of a pheromone receptor which comprises contacting the transfected cells or membrane fractions of the above described transfected cells with the compound under conditions permitting the activation of a functional pheromone receptor response, the activation of the receptor indicating that the compound is capable of activating the activity of a pheromone receptor.

This invention provides a method of identifying a compound capable of activating the activity of a pheromone receptor which comprises contacting a purified pheromone receptor with the compound under conditions permitting the activation of a functional pheromone receptor response, the activation of the receptor indicating that the compound is capable of activating the activity of a pheromone receptor.

This invention provides a method of identifying a compound capable of inhibiting the activity of a pheromone receptor which comprises contacting the transfected cells or membrane fractions of the above described transfected cells with an appropriate amount of the compound under conditions permitting the inhibition of a functional pheromone receptor response, the inhibition of the receptor response indicating that the compound is capable of inhibiting the activity of a pheromone receptor.

This invention provides a method of identifying a compound capable of inhibiting the activity of a pheromone receptor which comprises contacting an appropriate amount of the purified pheromone receptor with an appropriated amount of the compound under conditions permitting the inhibition of a functional pheromone receptor response, the inhibition of the receptor response indicating that the compound is capable of activating the activity of a pheromone receptor. In an embodiment of the above method, the purified receptor is embedded in a lipid bilayer.

This invention also provides compounds identified by the above methods. This invention further provides a pharmaceutical composition comprising an effective amount. of the identified compound and a pharmaceutically acceptable carrier.

This invention provides the use of the above compound for the preparation of a pharmaceutical composition for manipulating the maternal behavior of a female subject. In an embodiment, the female subject is a human.

This invention provides the use of the above compound for the preparation of a pharmaceutical composition for manipulating the social behavior of a subject. In an embodiment, the subject is a human. In another embodiment, the subject is an animal.

This invention provides the use of the above compound for the preparation of a pharmaceutical composition for manipulating the reproductive functions of an animal.

This invention provides the use of the above compound for the preparation of a pharmaceutical composition for manipulating the reproductive behaviors of a subject.

This invention provides the use of the above compound for the preparation of a pharmaceutical composition for increasing the fertility of a subject. This invention provides the use of the above compound for the preparation of a pharmaceutical composition for manipulating hormonal secretion of a subject to the subject.

This invention provides the use of the above compound for the preparation of a pharmaceutical composition for manipulating food intake rate of a subject.

The above different uses of the identified compounds are applicable to different domestic animals as. well as human.

This invention provides a composition for manipulating the maternal behavior of a female subject comprising effective amount of the identified compound and an acceptable carrier. This invention also provides a composition for manipulating the social behavior of a subject comprising effective amount of the identified compound and an acceptable carrier. This invention further provides a composition for manipulating the reproductive functions of a subject comprising effective amount of the identified compound and an acceptable carrier. This invention also provides a composition for changing the reproductive behavior of an animal comprising effective amount of the identified compound and an acceptable carrier. This invention provides a composition -for increasing the fertility of a subject comprising effective amount of the identified compound and an acceptable carrier.

This invention provides a composition for changing hormonal secretion of a subject comprising effective amount of the identified compound and an acceptable carrier.

In an embodiment, the compound is a polypeptide.

This invention provides a transgenic nonhuman living organism expressing DNA encoding a pheromone receptor, either the natural or modified form, and transgenic nonhuman living organism expressing DNA encoding the polypeptide capable of activating or inhibiting the activity of a pheromone receptor.

This invention also provides a transgenic nonhuman living organism comprising a homologous recombination knockout of the native pheromone receptor.

### Brief Description of the Figures

- Figure 1.: Spatial segregation of the vomeronasal organ and the main olfactory systems. (A) A drawing of a parasagittal section through the skull of a rat. The convoluted turbinates of the main olfactory system (MOE) reside within the posterior recess of the nasal cavity, whereas the vomeronasal organ (VNO) resides more anteriorly in a blind-ended pouch within the septum of the nose. The axons from sensory neurons of the MOE project to the main olfactory bulb (OB) , whereas the neurons of the VNO send axons to the anatomically distinct, more posteriorally-placed accessory olfactory bulb (AOB). (B) A drawing of a coronal section showing the anatomically distinct vomeronasal organ and the main olfactory epithelium. NC, nasal cavity; P, palate.

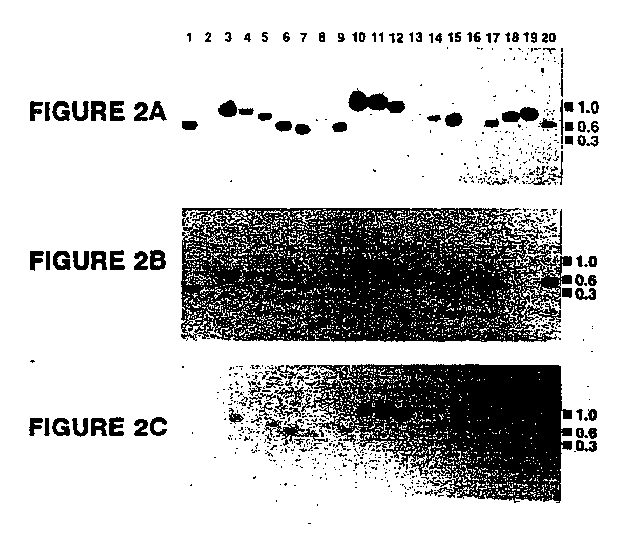

- Figure 2.: Identification of cDNA clones specifically expressed in an individual VNO neuron. 20 cDNA clones initially identified by differential screening of a cDNA library from a single VNO neuron were isolated. The inserts were amplified by PCR, electrophoresed on 1% agarose gels, and blotted to nylon filters. Blots were annealed with ³²P-labeled cDNA probe from VNO neuron 1 (A), VNO neuron 2 (B), or a neuron from the main olfactory epithelium (C). Two cDNA clones (18 and 19) only anneal with cDNA prepared from VNO neuron 1. One clone (17) anneals with the cDNA from both VNO neurons, but not with cDNA from an MOE neuron.

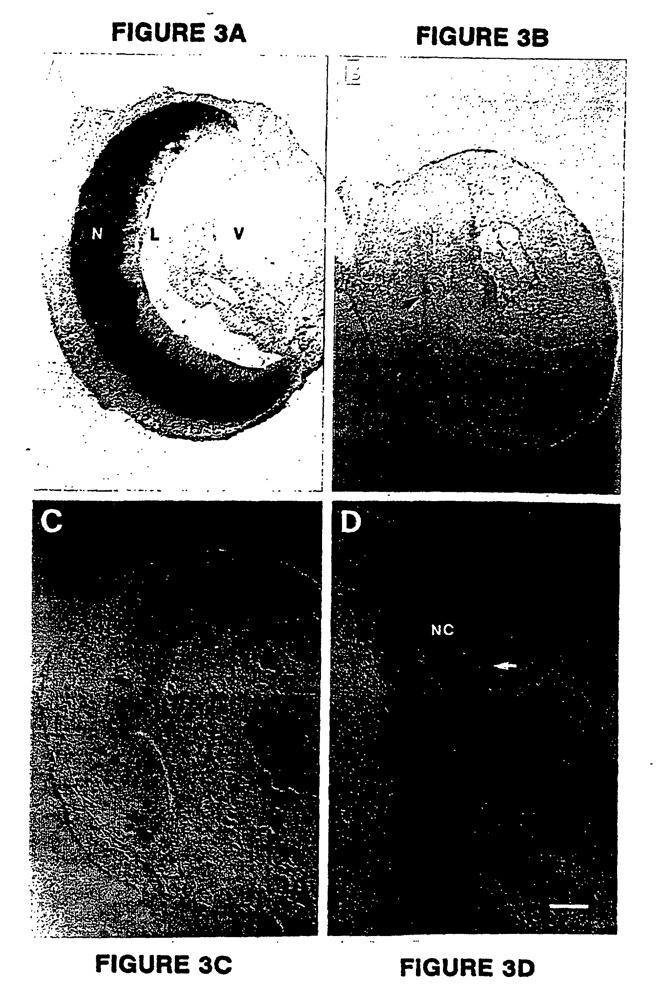

- Figure 3.: Expression of VN1 receptor RNA is restricted to a subset of vomeronasal neurons. Coronal sections of the vomeronasal organ dissected from adult male rats were annealed with digoxigenin-labeled, antisense RNA probes for (A) the olfactory marker protein (OMP); (B) The M12 receptor (a receptor expressed in abundance in the main olfactory epithelium) and (C) The VNO-specific receptor, VN1. (D) *In situ* hybridization of VN1 to a coronal section of turbinates from the newborn main olfactory epithelium. The arrow in B indicates a single positive VNO neuron expressing the MOE receptor, M12. In Panel A, N denotes the neuroepithelium; L, the lumen of VNO; and V the vomeronasal vein. In Panel D, the arrow points to the MOE; NC, nasal cavity. Scale bar equals 120mm

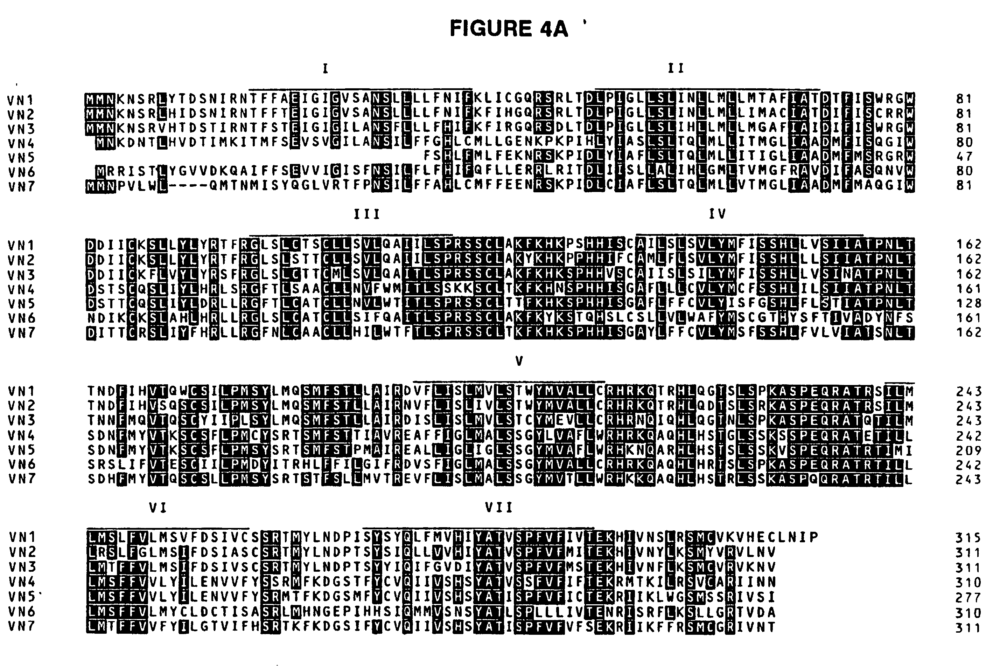

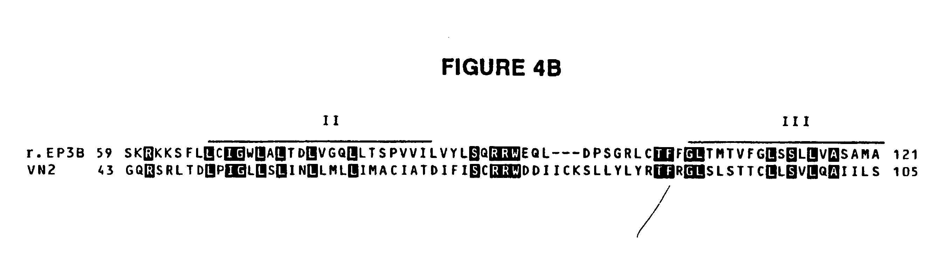

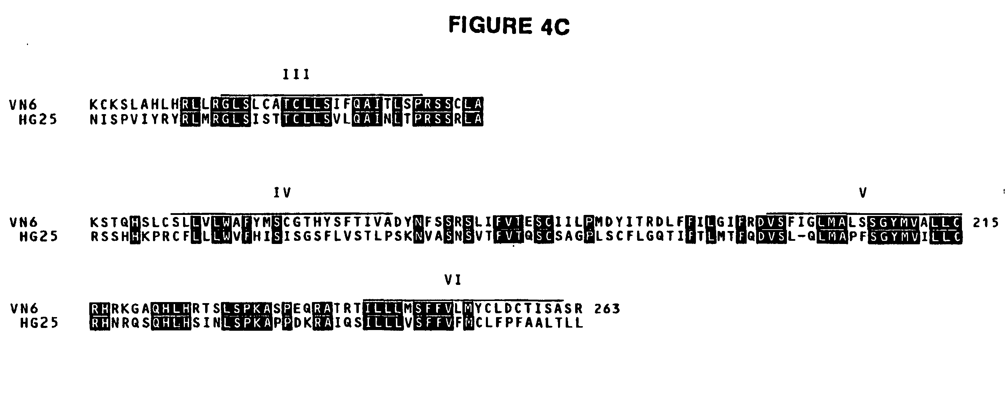

- Figure 4.: Deduced amino acid sequences of the pheromone receptor cDNAs. (A) The deduced amino acid sequences of seven putative pheromone receptor cDNAs, VN1, VN2, VN3, VN4, VN5, VN6, VN7 (Seq. ID. Nos.:8-14) are aligned. Predicted positions of the seven transmembrane domains are indicated (I-VIII. Amino acid residues common to at least five of the seven sequences are highlighted in black. (B) An alignment between the sequences of the second and third transmembrane domains of the rat prostaglandin receptor E3 (rEP3B) (Seq. ID. No.:16), and the VNO receptor VN2 (Seq. ID. No.:17) showing 28% identity over this region of the receptor sequence. (C) An alignement between the sequences of VN6 (Seq. ID. No.:18) and HG25 (Seq. ID. No.:15) which is deduced from a human clone.

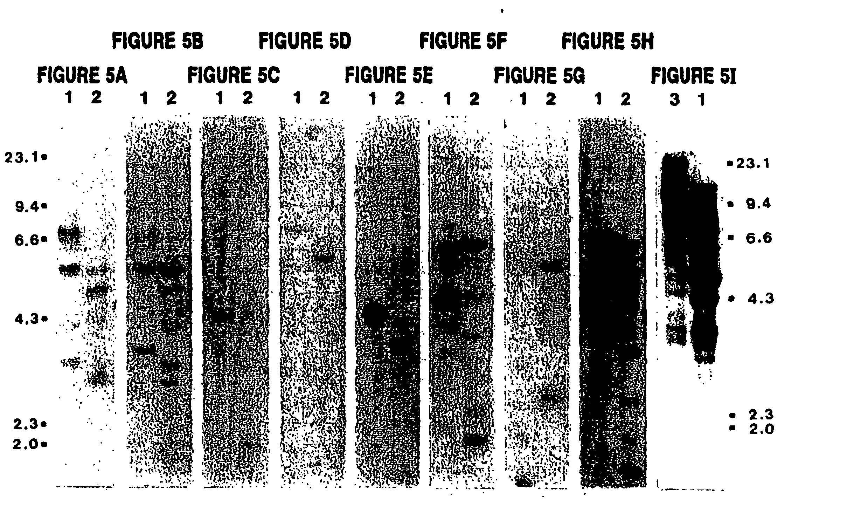

- Figure 5.: Southern blot analysis with the seven pheromone receptor cDNAs. Rat genomic DNA isolated from liver was digested with Pst1 (lanes 1) or EcoR1 (lanes 2), electrophoresed on 0.8% agarose gels, and blotted to nylon filters. Blots were annealed with ³²P-labled probes corresponding to the seven different receptor cDNAs, VN1-VN7 (Panels A-G, respectively). Under the high stringency conditions of hybridization and washing used in these experiments, cross hybridization is observed between VN1 and VN2, whereas the other individual receptor probes do not crosshybridize. A mix of six probes specific for each of the six receptor subfamilies (VN2-VN7) was annealed under conditions of high (H) and lower (I) stringency to either Pst1 (lanes 1), EcoR1 (lane 2), or Hind3 (lane 3) cleaved DNA (See Experimental Procedures). Panel I was run separately from Panels A-H, which were electrophoresed on the same gel.

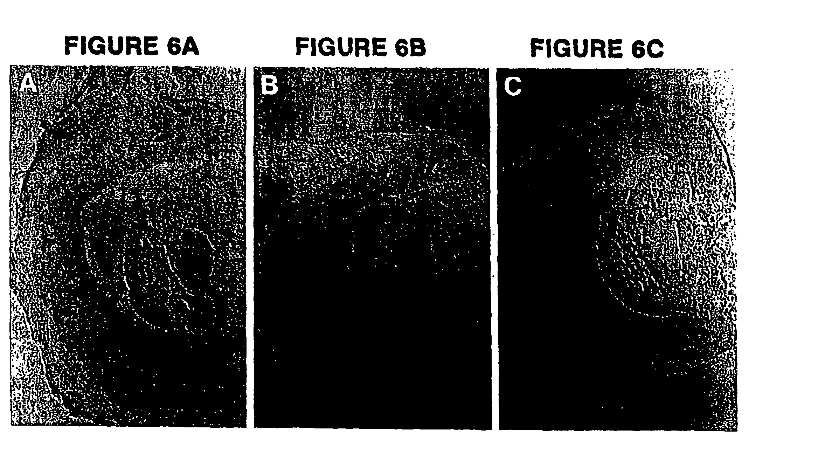

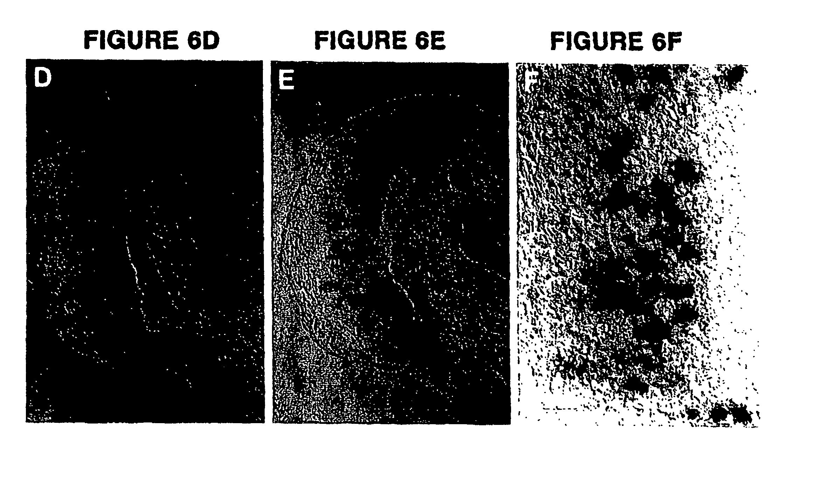

- Figure 6.: Localization of the individual receptors to distinct subpopulations of cells within the vomeronasal organ. *In situ* hybridization to coronal sections of a dissected VNO using digoxigenin-labeled probes from either the individual receptors, or a mix of the six receptors. Digoxigenin-labeled antisense RNA probes from receptor VN1 (A), receptor VN3 (C), receptor VN4 (D), or a mix of six probes specific for each receptor subfamilies (E) were annealed to a coronal section of the VNO dissected from male rats. Panel B shows the annealing of receptor VN1 probe to a section through the VNO from a female rat. Panel F shows a high power magnification of (E). VNO cDNA clones 1-7 label 2.7, 3.8, 1.1, 1.2, 1.1, 1.5, and 3% of the cells in the neuroepithelium, respectively. The mix of seven probes label 15% of the cells. Scale bar equals 120 mm.
- Figure 7.: Receptor expression is restricted to VNO neurons. A coronal section through the head of an E17 rat shows hybridization of a mix of 6 receptor probes to neurons within the VNO (arrows), but not to neurons within the main olfactory epithelium nor to other tissues in the nose. NC, nasal cavity; S, septum. Scale bar equals 250 mm.Nucleic acid sequence of cDNA A. The underlined ATG is the initiated codon used and the underlined TAA is the termination codon used.

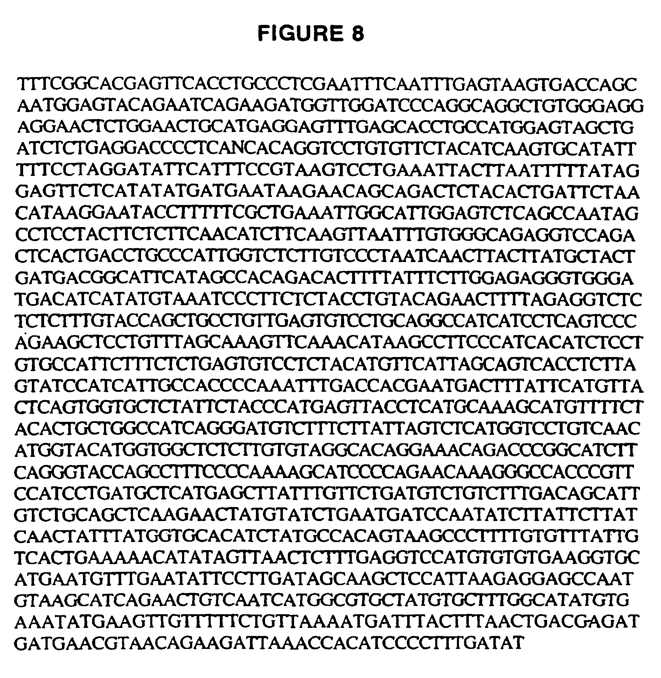

- Figure 8.: Nucleic acid sequence of VN1 (Seq. ID. No.:2).

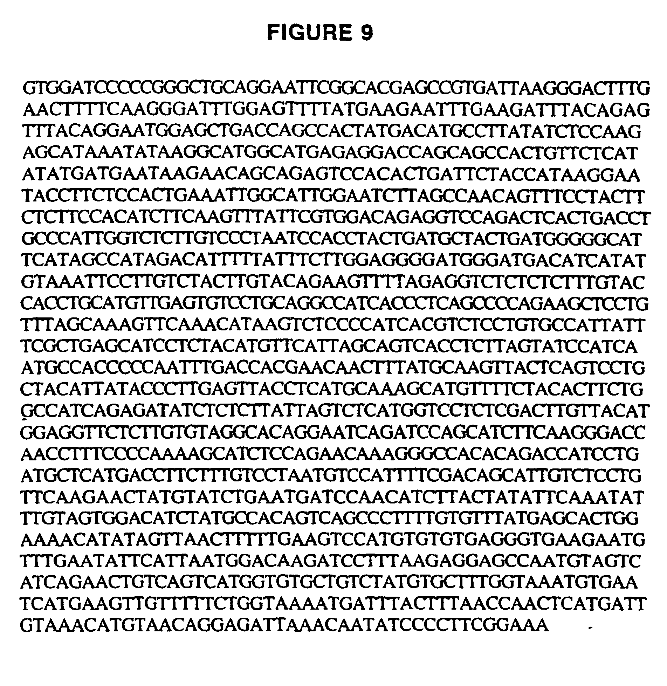

- Figure 9.: Nucleic acid sequence of VN3 (Seq. ID. No.:3).

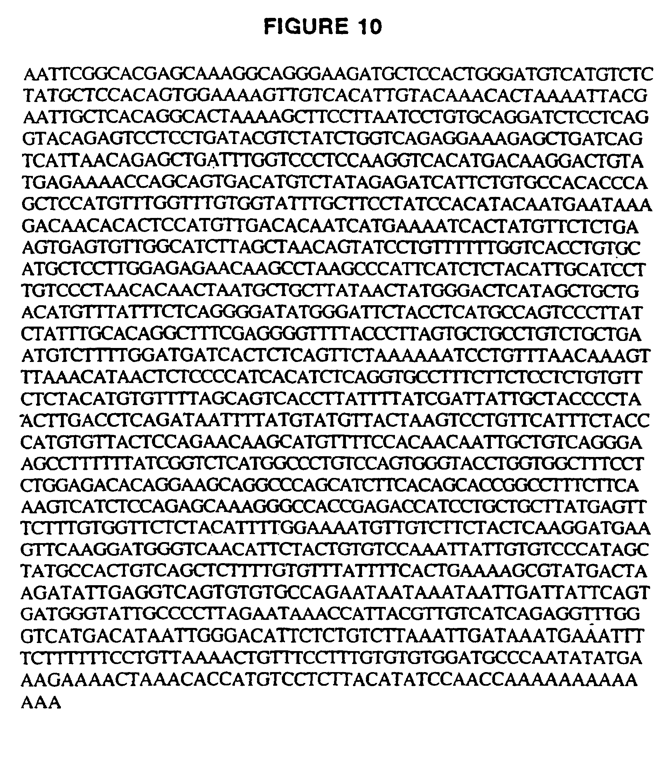

- Figure 10.: Nucleic acid sequence of VN4 (Seq. ID. No.:4).

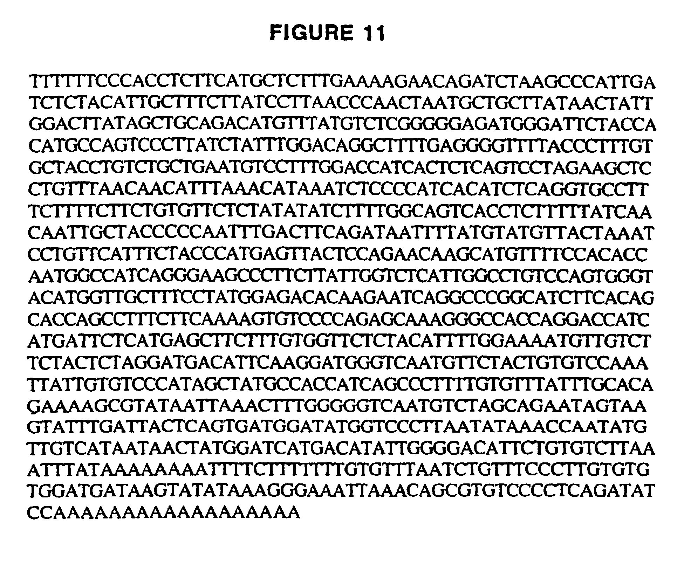

- Figure 11.: Nucleic acid sequence of VN5 (Seq. ID. No.:5).

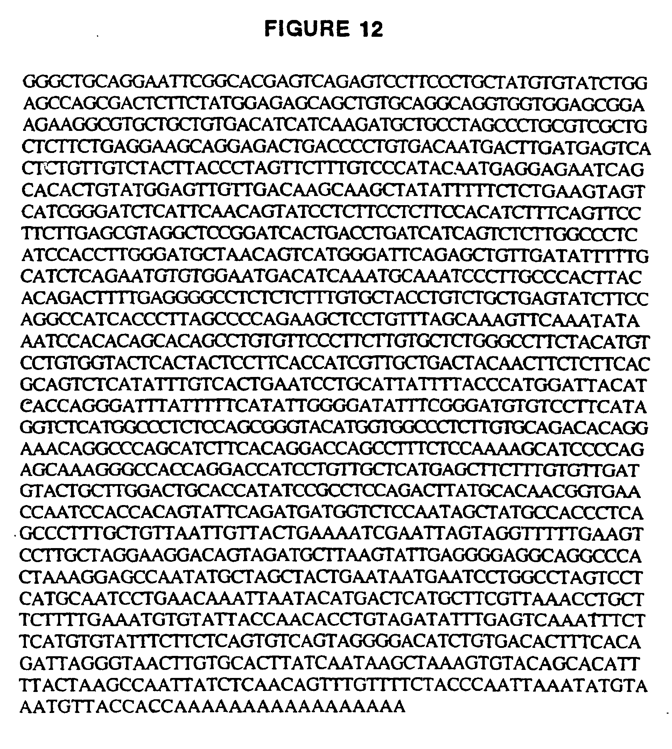

- Figure 12.: Nucleic acid sequence of VN6 (Seq. ID. No.:6).

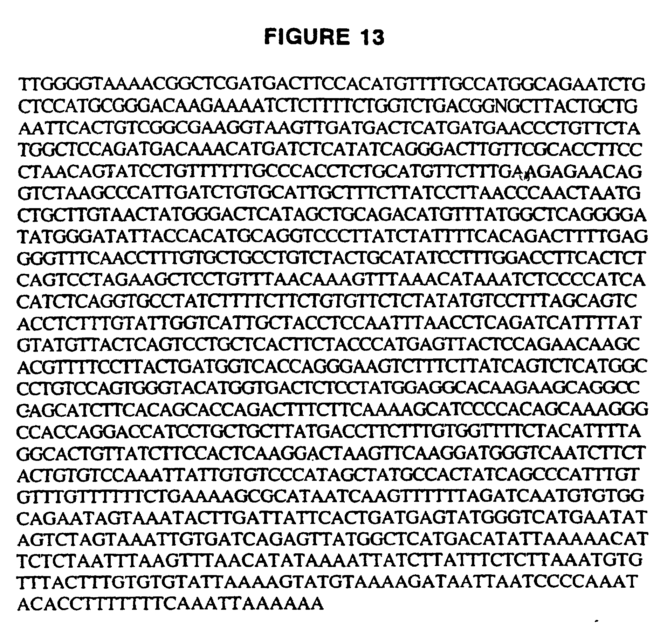

- Figure 13.: Nucleic acid sequence of VN7 (Seq. ID. No.:7).
- Figure 14.: Nucleic acid sequence of hg25x(Seq. ID. No.:1).

### Detailed Description of the Invention

This invention provides an isolated nucleic acid molecule encoding a vertebrate pheromone receptor. In an embodiment, the nucleic acid molecule is a DNA molecule. The DNA may be cDNA, genomic or synthetic DNA. In another embodiment, the nucleic acid is an RNA molecule.

In a further embodiment, the nucleic acid molecule encodes a mammalian pheromone receptor. In a still further embodiment, the nucleic acid molecule encodes a rat pheromone receptor. In another further embodiment, the nucleic acid molecule encodes a human pheromone receptor.

The nucleic acid molecules encoding a pheromone receptor includes molecules coding for polypeptide analogs, fragments or derivatives of antigenic polypeptides which differ from naturally-occurring forms in terms of the identity or location of one or more amino acid residues (deletion analogs containing less than all of the residues specified for the protein, substitution analogs wherein one or more residues specified are replaced by other residues and addition analogs where in one or more amino acid residues is added to a terminal or medial portion of the polypeptides) and which share some or all properties of naturally-occurring forms.

These molecules include but not limited to: the incorporation of codons "preferred" for expression by selected non-mammalian hosts; the provision of sites for cleavage by restriction endonuclease enzymes; and the provision of additional initial, terminal or intermediate sequences that facilitate construction of readily expressed vectors. Accordingly, these changes may result in a modified pheromone receptor. It is the intent of this invention to include nucleic acid molecules which encodes modified pheromone receptor. Also, to facilitate the expression of receptor in different host cells, it may be necessary to modify the molecule such that the expressed receptors may reach the surface of the host cells. The modified pheromone receptor should have biological activities similar to the unmodified pheromone receptor. The molecules may also be modified to increase the biological activity of the expressed receptor.

This invention also provides a nucleic acid molecule of at least 12 nucleotides capable of specifically hybridizing with a unique sequence within the sequence of the above-described nucleic acid molecule. This nucleic acid molecule may be DNA or RNA.

This invention provides a vector which comprises the above-described isolated nucleic acid molecule.

In another embodiment, the vector is a plasmid. In a further embodiment, the plasmid is designated VN1 (ATCC Accession No.97294). In another embodiment, the plasmid is designated VN3 (ATCC Accession No. 97295). In a separate embodiment, the plasmid is designated VN4 (ATCC Accession No.97296). In another embodiment, the plasmid is designated VN5 (ATCC Accession No.97297). In a separate embodiment, the plasmid is designated VN6 (ATCC Accession No.97298). In a still further embodiment, the plasmid is designated VN7 (ATCC Accession No. 97299).

Plasmids VN1, VN3, VN4, VN5, VN6 and VN7 were made by cloning the DNA inserts which encoding a pheromone receptor into the XhoI and EcoRI sites of the plasmid pBluescript. VN1, VN3, VN4, VN5, VN6 and VN7 were deposited on September 27, 1995 with the American Type Culture Collection (ATCC), 12301 Parklawn Drive, Rockville, Maryland 20852, U.S.A. under the provisions of the Budapest Treaty for the International Recognition of the Deposit of Microorganism for the Purposes of Patent Procedure. The plasmids, VN1, VN3, VN4, VN5, VN6 and VN7 were accorded ATCC Accession Numbers 97294-97299.

The sequences of the DNA inserts of VN1, VN3, VN4, VN5, VN6 and VN7 were submitted to GenBank and were assigned with accession numbers U36785, U36895, U36896, U36897, U36898, and U36786 respectively. VN2 was assigned with GenBank accession number U36899.

In an embodiment, the above described isolated nucleic acid molecule is operatively linked to a regulatory element.

Regulatory elements required for expression include promoter sequences to bind RNA polymerase and transcription initiation sequences for ribosome binding. For example, a bacterial expression vector includes a promoter such as the lac promoter and for transcription initiation the Shine-Dalgarno sequence and the start codon AUG. Similarly, a eukaryotic expression vector includes a heterologous or homologous promoter for RNA polymerase II, a downstream polyadenylation signal, the start codon AUG, and a termination codon for detachment of the ribosome. Such vectors may be obtained commercially or assembled from the sequences described by methods well-known in the art, for example the methods described above for constructing vectors in general.

This invention provides a host vector system for the production of a polypeptide having the biological activity of a vertebrate pheromone receptor which comprises the above-described vector and a suitable host.

This invention also provides a host vector system, wherein the suitable host is a bacterial cell, yeast cell, insect cell, or animal cell. The host cell of the above expression system may be selected from the group consisting of the cells where the protein of interest is normally expressed, or foreign cells such as bacterial cells (such as *E. coli*), yeast cells, fungal cells, insect cells, nematode cells, plant or animal cells, where the protein of interest is not normally expressed. Suitable animal cells include, but are not limited to Vero cells, HeLa cells, Cos cells, CV1 cells and various primary mammalian cells.

This invention provides a method of producing a polypeptide having the biological activity of a vertebrate pheromone receptor which comprising growing the above-described host vector system under conditions permitting production of the polypeptide and recovering the polypeptide so produced.

This invention also provides a purified, vertebrate pheromone receptor.

This invention also provides a polypeptide encoded by the above-described isolated vertebrate nucleic acid molecule.

This invention provides an antibody capable of binding to a vertebrate pheromone receptor. This invention further provides an antibody capable of competitively inhibiting the binding of the antibody capable of specifically binding to a vertebrate pheromone receptor. In an embodiment, the antibody is monoclonal. In another embodiment, the antibody is polyclonal.

Monoclonal antibody directed to a pheromone receptor may comprise, for example, a monoclonal antibody directed to an epitope of a pheromone receptor present on the surface of a cell. Amino acid sequences may be analyzed by methods well known to those skilled in the art to determine whether they produce hydrophobic or hydrophilic regions in the proteins which they build. In the case of cell membrane proteins, hydrophobic regions are well known to form the part of the protein that is inserted into the lipid bilayer which forms the cell membrane, while hydrophilic regions are located on the cell surface, in an aqueous environment.

Antibodies directed to a pheromone receptor may be serum-derived or monoclonal and are prepared using methods well known in the art. For example, monoclonal antibodies are prepared using hybridoma technology by fusing antibody producing B cells.from immunized animals with myeloma cells and selecting the resulting hybridoma cell line producing the desired antibody. Cells such as NIH3T3 cells or 293 cells which express the receptor may be used as immunogens to raise such an antibody. Alternatively, synthetic peptides may be prepared_using commercially available machines.

As a still further alternative, DNA, such as a cDNA or a fragment thereof, encoding the receptor or a portion of the receptor may be cloned and expressed. The expressed polypeptide recovered and used as an immunogen.

The resulting antibodies are useful to detect the presence of pheromone receptors or to inhibit the function of the receptor in living animals, in humans, or in biological tissues or fluids isolated from animals or humans.

This antibodies may also be useful for identifying or isolating other pheromone receptor. For example, antibodies against the rat pheromone receptor may be used to screen a human expression library for a human pheromone receptor. Such antibodies may be monoclonal or monospecific polyclonal antibody against a selected pheromone receptor. Human expression libraries are readily available and may be made using technologies well-known in the art.

This invention provides a method for identifying cDNA inserts encoding pheromone receptors comprising: (a) generating a cDNA library which contains clones carrying cDNA inserts from an individual vomeronasal sensory neuron;(b) hybridizing nucleic acid molecules of clones from the cDNA libraries generated in step (a) with probes prepared from the individual vomeronasal neuron and probes from a secondary individual vomeronasal neuron or a main olfactory epithelium neuron;(c) selecting clones which hybridized with probes from the individual vomeronasal neuron but not from the second individual vomeronasal neuron or the main olfactory epithelium neuron; and (d) isolating clones which carry the hybridized inserts, thereby identifying the inserts encoding pheromone receptors.

This invention also provides the above-described method wherein after step (c), further comprising: (a) amplifying the inserts from the selected clones by polymerase chain reaction; (b) hybridizing the amplified inserts with probes from the individual vomeronasal neuron; and-(c) isolating the clones which carry the hybridized inserts, thereby identifying the inserts encoding the pheromone receptors. The probes used may be cDNA .

This invention also provides cDNA inserts identified by the above methods.

This invention also provides a method for identifying DNA inserts encoding pheromone receptors comprising: (a) generating DNA libraries which contain clones carrying inserts from a sample containing at least one vomeronasal sensory neuron; (b) contacting clones from the cDNA libraries generated in step (a) with nucleic acid molecule of at least 12 nucleotides capable of specifically hybridizing with a unique sequence within the sequence of a pheromone receptor in appropriate conditions permitting the hybridization of the nucleic acid molecules of the clones and the nucleic acid molecule; (c) selecting clones which hybridized with nucleic acid molecule; and (d) isolating the clones which carry the hybridized inserts, thereby identifying the inserts encoding the pheromone receptors. In an embodiment, the sample contain only one individual vomeronasal sensory neuron.

One means of isolating a nucleic acid molecule which encodes a pheromone receptor is to probe a libraries with a natural or artificially designed probes, using methods well known in the art. The probes may be DNA or RNA. The library may be cDNA or genomic DNA.

This invention also provides a method to identify DNA inserts encoding pheromone receptors comprising: (a) generating DNA libraries which contain clones with inserts from-a sample containing at least one vomeronasal sensory neuron; (b) contacting the clones from the DNA libraries generated in step (a) with appropriate polymerase chain reaction primers capable of specifically binding to nucleic acid molecules encoding pheromone receptors in appropriate conditions permitting the amplification of the hybridized inserts by polymerase chain reaction; (c) selecting the amplified inserts; and (d) isolating the amplified inserts, thereby identifying the inserts encoding the pheromone receptors. In an embodiment, the sample contains only one individual vomeronasal sensory neuron. In a separate embodiment of the above methods, the libraries are cDNA libraries. In another embodiment, the libraries are cDNA libraries.

The appropriate polymerase chain reaction primers may be chosen from the conserved regions of the known pheromone receptor sequences. Alternatively, the primers may be chosen from the regions which are the active sites for the binding of ligands.

This invention also provides DNA inserts identified by the above methods.

This invention provides a method to isolate DNA molecules encoding pheromone receptors comprising: (a) contacting a biological sample known to contain nucleic acids with appropriate polymerase chain reaction primers capable of specifically binding to nucleic acid molecules encoding pheromone receptors in appropriate conditions permitting the amplification of the hybridized molecules by polymerase chain reaction; (b) isolating the amplified molecules, thereby identifying the DNA molecules encoding the pheromone receptors. In an embodiment, the sample contains DNA. In another embodiment, the sample contains genomic DNA.-

This invention also provides the nucleic acid molecules isolated by the above method.

This invention provides a method of transforming cells which comprises transfecting a host cell with a suitable vector comprising a nucleic acid molecule encoding a pheromone receptor as described above. This invention also provides transformed cells produced by above method.

The invention also provides transformed cells wherein the host cells are not usually expressing pheromone receptors and transformed cells wherein the host cells are expressing pheromone receptors.

This invention provides a method of identifying a compound capable of specifically binding to a vertebrate pheromone receptor which comprises contacting a transfected cells or membrane fractions of the above transfected cells with an appropriate amount of the compound under conditions permitting binding of the compound to such receptor, detecting the presence of any such compound specifically bound to the receptor, and thereby determining whether the compound specifically binds to the receptor.

This invention provides a method of identifying a compound capable of specifically binding to a vertebrate pheromone receptor which comprises contacting an . appropriate amount of the purified pheromone receptor with an appropriate amount of the compound under conditions permitting binding of the compound to such purified receptor, detecting the presence of any such compound specifically bound to the receptor, and thereby determining -whether the compound specifically binds to the receptor.

In an embodiment, the purified receptor is embedded in a lipid bilayer. The purified receptor may be embedded in the liposomes with proper orientation to carry out normal functions. Liposome technology is well-known in the art.

This invention also provides a method of identifying a compound capable of activating the activity of a pheromone receptor which comprises contacting the transfected cells or membrane fractions of the above described transfected cells with the compound under conditions permitting the activation of a functional pheromone receptor response, the activation of the receptor indicating that the compound is capable of activating the activity of a pheromone receptor.

This invention provides a method of identifying a compound capable of activating the activity of a pheromone receptor which comprises contacting a purified pheromone receptor with the compound under conditions permitting the activation of a functional pheromone receptor response, the activation of the receptor indicating that the compound is capable of activating the activity of a pheromone receptor. In an embodiment, the purified receptor is embedded in a lipid bilayer. As discussed hereinabove, the purified receptors may be embedded in liposomes with proper orientations to carry out their normal functions.

This invention provides a method of identifying a compound capable of inhibiting the activity of a pheromone receptor which comprises contacting the transfected cells or membrane fractions of the above described transfected cells with an appropriate amount of the compound under conditions permitting the inhibition of a functional pheromone receptor response, the inhibition of the receptor response indicating that the compound is capable of inhibiting the activity of a pheromone receptor.

This invention provides a method of identifying a compound capable of inhibiting the activity of a pheromone receptor which comprises contacting an appropriate amount of the purified pheromone receptor with an appropriated amount of the compound under conditions permitting the inhibition of a functional pheromone receptor response, the inhibition of the receptor response indicating that the compound is capable of activating the activity of a pheromone receptor. In an embodiment of the above method, the purified receptor is embedded in a lipid bilayer.

In another embodiment of the above methods, the compound used is not previously known.

This invention also describes compounds identified by the above methods. This invention further provides a pharmaceutical composition comprising an effective amount of the identified compound and a pharmaceutically acceptable carrier.

Pharmaceutically acceptable carriers are well known to those skilled in the art and include, but are not limited to, 0.01-0.1M and preferably 0.05M phosphate buffer or 0.8% saline. Additionally, such pharmaceutically acceptable carriers may be aqueous or non-aqueous solutions, suspensions, and emulsions. Examples of non-aqueous solvents are propylene glycol, polyethylene glycol, vegetable oils such as olive oil, and injectable organic esters such as ethyl oleate. Aqueous carriers include water, alcoholic/aqueous solutions, emulsions or suspensions, including saline and buffered media. Parenteral vehicles include sodium chloride solution, Ringer's dextrose, dextrose and sodium chloride, lactated Ringer's or fixed oils. Intravenous vehicles include fluid and nutrient replenishers, electrolyte replenishers such as those based on Ringer's dextrose, and the like. Preservatives and other additives may also be present, such as, for example, antimicrobials, antioxidants, chelating agents, inert gases and the like.

This invention provides the use of the above compound for the preparation of a pharmaceutical composition for manipulating the maternal behavior of a female subject. In an embodiment, the female subject is a human. In another embodiment, the subject is an animal.

This invention provides the use of the above compound for the preparation of a pharmaceutical composition for manipulating the social behavior of a subject. In an embodiment, the subject is a human. In another embodiment, the subject is an animal.

This invention provides the use of the above compound for the preparation of a pharmaceutical composition for manipulating the reproductive functions of a subject.

This invention provides the use of the above compound for the preparation of a pharmaceutical composition for manipulating the reproductive behavior of a subject.

This invention provides the use of the above compound for the preparation of a pharmaceutical composition for increasing the fertility of a subject.

This invention provides the use of the above compound for the preparation of a pharmaceutical composition for manipulating hormonal secretion of a subject. In an embodiment, the hormone is the luteinizing hormone release hormone. In another embodiment, the hormone is the luteinizing hormone. In a further embodiment, the hormone is the prolactin release hormone. In a still further embodiment, the hormone is prolactin.

This invention provides the use of the above compound for the preparation of a pharmaceutical composition for changing food intake rate of a subject.

The above different uses of the identified compounds are applicable to different animals as well as human.

This invention provides a composition for manipulating the maternal behavior of a female subject comprising effective amount of the above compound and an acceptable carrier. This invention also provides a composition for manipulating the social behavior of a subject comprising effective amount of the above compound and an acceptable carrier. This invention further provides a composition. for manipulating the reproductive functions of a subject comprising effective amount of the above compound. This invention also provides a composition for changing the reproductive behavior of an animal comprising effective amount of the above compound. This invention provides a composition for increasing the fertility of an animal comprising effective amount of the above compound and an acceptable carrier. The subject may be human or animal.

This invention provides a composition for manipulating hormonal secretion of a subject comprising effective amount of the above compound and an acceptable carrier. In an embodiment, the hormone is the luteinizing hormone release hormone. In another embodiment, the hormone is the luteinizing hormone. In a further embodiment, the hormone is the prolactin release hormone. In a still further embodiment, the hormone is the prolactin. The subject may be human or animal.

As it is well known in the art, various carriers may be used according to this invention. For example, the compound may dissolve in physiological saline for administration to animal or human.

In an embodiment, the compound is a polypeptide.

This invention provides a transgenic nonhuman living organism expressing DNA encoding a vertebrate pheromone receptor. This invention also provides a transgenic nonhuman living organism expressing DNA encoding the polypeptide which is capable of inhibiting the activity of a pheromone receptor. In an embodiment, the living organism is a transgenic animal.

This invention also provides a transgenic nonhuman living organism comprising a homologous recombination knockout of the native pheromone receptor. In an embodiment, the transgenic is an animal.

One means available for producing a transgenic animal, with a mouse as an example, is as follows: Female mice are mated, and the resulting fertilized eggs are dissected out of their oviducts. The eggs are stored in an appropriate medium such as M2 medium (Hogan B. et al. Manipulating the Mouse Embryo, A Laboratory Manual, Cold Spring Harbor Laboratory (1986)). DNA or cDNA encoding a pheromone receptor is purified from a vector by methods well known in the art. Inducible promoters may be fused with the coding region of the DNA to provide an experimental means to regulate expression of the trans-gene. Alternatively or in addition, tissue specific regulatory elements may be fused with the coding region to permit tissue-specific expression of the trans-gene. The DNA, in an appropriately buffered solution, is put into a microinjection needle (which may be made from capillary tubing using a pipet puller) and the egg to be injected is put in a depression slide. The needle is inserted into the pronucleus of the egg, and the DNA solution is injected. The injected egg is then transferred into the oviduct of a pseudopregnant mouse (a mouse stimulated by the appropriate hormones to maintain pregnancy but which is not actually pregnant), where it proceeds to the uterus, implants, and develops to term. As noted above, microinjection is not the only method for inserting DNA into the egg cell, and is used here only for exemplary purposes.

This invention will be better understood from the Experimental Details which follow. However, one skilled in the art will readily appreciate that the specific methods and results discussed are merely illustrative of the invention as described more fully in the claims which follow thereafter.

### Experimental Details

### Experimental Strategy

Applicants' initial efforts to identify the genes encoding the pheromone receptors were based upon the assumption that the main olfactory epithelium and the vomeronasal organ might share a common evolutionary origin such that DNA sequence homology may exist between the two receptor families. However, low stringency hybridization of MOE receptor probes to rat vomeronasal cDNA libraries, as well as polymerase chain reactions (PCR) using conserved motifs from both the family of odorant receptor genes, as well as from the superfamily of known seven transmembrane domain receptors were consistently unsuccessful. Moreover, the components of the olfactory signal transduction cascade in the main olfactory epithelium [(the olfactory-specific G-protein, G_{olf}, (Jones and Reed, 1989), the olfactory-specific adenylate cyclase (Bakalyar and Reed, 1990), and the cyclic nucleotide responsive ion channel (Dhallan et al., 1990; Ludwig et al., 1990)] were not detectable in VNO neurons by *in situ* hybridization or by screening cDNA libraries (data not shown). These observations suggested that the pheromone receptors and the signal transduction pathways which they activate might have evolved independently in the VNO and the MOE.

Applicants therefore developed a cloning procedure that made no assumptions concerning the structural class of the receptor molecules. Rather, applicants only assumed that the expression of the pheromone receptors would be restricted to the vomeronasal organ, and that individual neurons within the VNO were likely to express different receptor genes. In the main olfactory epithelium, about 1% of the mRNA in a given sensory cell encodes a given receptor (Vassar et al., 1994). However, the thousand different receptor genes are each expressed in different neurons such that the frequency of a specific receptor RNA will be diluted to 0.001% of the mRNA message population. The generation of libraries from individual neurons provided an experimental solution to the problem of detecting a specific mRNA in a heterogeneous population of neurons. RT-PCR was therefore used to generate double-stranded cDNA, as well as cDNA libraries from individual vomeronasal sensory neurons. Applicants expected that the frequency of a specific receptor cDNA in libraries from single neurons would be about 1%. Differential screening of such libraries from single neurons should therefore permit the isolation of pheromone receptor genes.

In control experiments, the cDNA library prepared from a single rat VNO neuron was screened with probes for tubulin and olfactory marker protein (OMP) to determine whether these libraries accurately represent the mRNA population. The frequency of these clones suggested that the representation of a given RNA was not biased in the construction of the library (see Experimental Procedures). One thousand recombinant phages from a cDNA library prepared from VNO neuron 1 were then screened in triplicate with cDNA probes prepared from VNO neuron 1, a second VNO neuron (VNO neuron 2), and a neuron from the MOE. About 2% of the cDNA clones screened showed specific hybridization with cDNA probes from VNO neuron 1, but not with probes from VNO neuron 2 or the MOE neuron. The specificity of these cDNA clones was further examined in a more sensitive assay. The inserts from these cDNA clones were amplified by PCR and the DNA products were hybridized on Southern blots with cDNA probes from VNO neuron 1, VNO neuron 2, or from an MOE sensory neuron (Figure 2). Of 20 clones initially isolated from the VNO neuron 1 cDNA library, only two (clones 18 and 19 in figure 2A) appeared to be specific to VNO neuron 1 in this more sensitive screen. These two clones represented independent isolates of an identical cDNA sequence present within the cDNA library of VNO neuron 1 at a frequency of 0.5%. This cDNA was used as a probe to isolate full-length clones from a cDNA library with larger inserts constructed with RNA prepared from several dissected vomeronasal organs. A full length clone, VN1 encodes a seven transmembrane domain receptor (see below).

The pattern of expression of this cDNA was determined by performing RNA *in situ* hybridization to sections through the rat vomeronasal organ. In cross section, a thick multicellular sensory epithelium lines half of the lumen of the vomeronasal organ (Figure 3). *In situ* hybridization demonstrates that mature VNO neurons uniformly express the olfactory marker protein (OMP) (Figure 3A). In contrast, the cDNA specific for VNO neuron 1 localized to a subpopulation of VNO neurons (Figure 3C). No hybridization was observed in the MOE (Figure 3D), or in any other neural or non-neural cells (see below).

Thus, difference cloning from libraries prepared from single neurons has allowed the isolation of a novel seven transmembrane domain receptor expressed in VNO sensory neurons.

### The Sequence of Several Members of the Receptor Gene Family

Applicants observed that VN1 is expressed in about 4% of the vomeronasal sensory neurons. This suggested the existence of a gene family with individual member genes expressed in different subsets of neurons. Applicants therefore used both PCR and high and low stringency hybridization to VNO cDNA libraries to identify possible members of a receptor gene family expressed in other VNO neurons (see Experimental Procedures). The sequences of seven different cDNAs obtained in this manner are aligned in Figure 4. Hydropathy analysis suggests that each of the seven sequences contain seven hydrophobic stretches that represent potential transmembrane domains. Sequence analysis suggests that these putative receptors are likely to adopt a structure similar to that of the previously characterized superfamily of seven transmembrane receptors. However, the VNO receptors do not share any of the conserved sequence motifs exhibited by members of the previously identified superfamily (Baldwin, 1993; Probst et al., 1992). One region of homology however is observed with the family of mammalian prostaglandin receptors throughout the second and third transmembrane domains (Figure 4B). Twenty-five percent identity is observed between VN2 and the rat E3 prostaglandin receptor over these two domains, but no significant sequence homology is observed in other regions of the molecule. Prostaglandins are potent pheromones eliciting mating in fish, but their role as mammalian pheromones is unknown. However, this level of homology over a small region of the protein does not permit us to argue that the receptors may recognize prostaglandins.

Overall, the seven VNO cDNA sequences share between 47% and 87% sequence identity. As observed previously for the odorant receptors from the MOE (Buck and Axel 1991), this family of VNO receptors exhibits significant divergence within the transmembrane domains, the presumed site of ligand binding (Strader et al., 1994). This pattern of divergence suggests that the different members may permit the binding of different structural classes of ligands.

### The Size of the Gene Family

Applicants have analyzed the size of the vomeronasal receptor gene family by performing hybridizations to genomic DNA, as well as quantitative screening of genomic libraries. The seven cDNAs that applicants have characterized fall within six subfamilies as defined by the observation that no crosshybridization is observed among the different subfamilies under high stringency conditions. cDNA probes from each of the six subfamilies were then annealed to Southern blots of rat genomic DNA after digestion with two different restriction endonucleases (Figure 5). The vomeronasal receptor genes analyzed thus far do not contain introns within the coding region (data not shown). Restriction cleavage was performed with endonucleases that do not cleave within the cDNAs applicants have isolated such that the number of hybridizing bands will closely approximate the number of receptor genes. Probes from each of the subfamilies identified from two to eight bands in genomic DNA such that a total of about 20 bands were detected in hybridizations with the six individual probes. A mix of six probes identifies about 20 bands in genomic DNA at high stringency of hybridization (Figure 5H) and more than 30 bands using less stringent conditions (Figure 51).

An independent estimate of the size of the gene family was obtained by screening a genomic library. A mix of the seven cDNA clones was used as a hybridization probe under reduced stringency conditions to identify about 35 positive clones per haploid genome. Thus, the data from Southern blotting and screens of genomic library are in accord with one another and indicate that the multigene family of vomeronasal receptors applicants have identified consists of between 30 and 40 genes.

### The Pattern of Receptor Expression in the Vomeronasal Organ

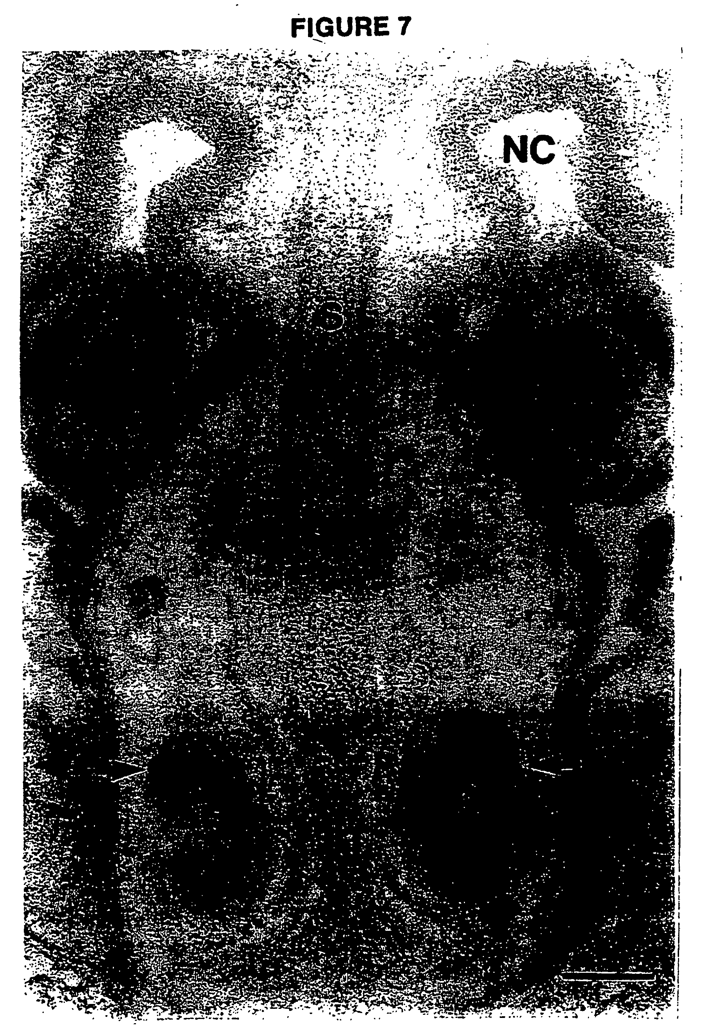

Applicants performed *in situ* hybridization to examine the spatial pattern of receptor expression in the sensory epithelium of the VNO. The VNO consists of a blind-ended tubular structure which extends in an anterior posterior dimension within the septum . In cross section, the sensory eipithelium lines the medial half of the tube and a vein surrounded by non-neuronal tissue resides more laterally (Figure 3A, and Figure 6). RNA *in situ* hybridization experiments were performed with digoxigenin-labeled RNA antisense probes from each of the six subfamilies under high stringency conditions, such that it was likely that a given probe will only detect members within its own subfamily. The results with each of the six probes were qualitatively indistinguishable. In each case, applicants observed a punctate distribution of cells expressing a given receptor RNA (Figure 6). No differences in the patterns of *in situ* hybridization were observed between males and females (Figure 6A and B) Each probe detected from about 1-4% of the VNO sensory neurons. These data contrast with hybridization patterns observed with the probe for the olfactory marker protein OMP (Figure 3A), which demonstrated uniform labeling of the VNO epithelium. Control sections hybridized with sense receptor probes revealed no specific signal (data not shown). Expression of this gene family was only observed in VNO neurons, no labelling was observed in the sensory neurons of the MOE (Figures 3D and 7). Hybridization of the M12 receptor from the MOE reveals a rare positive cell at a frequency of about 1 in 20,000 VNO neurons (Figure 3B). Finally, no expression of the VNO receptors was observed upon *in situ* hybridization to sections through brain, kidney, testes, and liver (data not shown).

Analysis of several sections through the entire VNO suggested that neurons expressing a given receptor are not topologically localized but rather are randomly distributed along the anterior-posterior axis. In cross section, however, neurons expressing the receptor family applicants have cloned are preferentially localized to the apical two thirds of the zone of OMP positive cells. Previous studies in the opossum have demonstrated that this apical zone of neurons expresses the G protein Gᵢ₂ₐ, whereas the more basal zone expresses Gₒ (Halpern et al., 1995), and a similar pattern is observed in rat (Belluscio, L., Dulac, C., and Axel, R. unpublished studies). Therefore, the expression of the family of receptors applicants have isolated may be restricted to Gᵢ₂ₐ positive cells. It is possible that the Gₒ positive cells express a more distant family of receptors.

### Individual Neurons Express Different Complements of Receptors.

In the main olfactory epithelium, a given neuron is likely to express only one receptor from the family of one thousand receptor genes. Moreover, neurons expressing a given receptor project their axons to one or a small number of topographically defined glomeruli within the olfactory bulb. The regulated expression of odorant receptors assuring that only one receptor is expressed in individual olfactory neurons is an important element in the coding of olfactory information in the main olfactory system. Quantitative analysis of the *in* *situ* hybridizations of the VNO receptor probes indicate that neurons within the VNO similarly express only a single receptor gene.

The observation that 1-4% of the VNO neurons express a given receptor subfamily suggests that each cell expresses only a subset of receptor genes. If applicants demonstrate that each of the different receptor probes hybridizes with distinct non-overlapping subpopulations of neurons, this would provide evidence that neurons differ with respect to the receptors they express. Sections were annealed with probes specific for each of the six receptor subfamilies, either individually or with a mixture of six probes (Figure 6). If each receptor is expressed in a distinct non-overlapping subpopulation of neurons, then the sum of the cells identified with the six probes should equal the number of cells identified with the mixed probe. In accord with this suggestion, applicants observe that the percentage of olfactory neurons detected with the mixed probe (15%) is significantly greater than the percentage detected with any of the individual probes alone, and approximates the sum of the percentage of positive neurons detected with the six individual probes (12%). These values are present in the legend to Figure 6. These results suggest that the six receptor subfamilies are expressed in distinct non-overlapping populations of olfactory neurons and provide support for a model in which a single sensory neuron expresses a single receptor gene.

### Experimental Discussion

Applicants have identified a novel family of seven transmembrane domain proteins which encode the mammalian pheromone receptors. Differential screening of cDNA libraries constructed from single sensory neurons initially led to the isolation of a family of putative receptor genes. Each member of the gene family is expressed in a small subpopulation of neurons such that the seven putative receptor genes applicants have cloned identify 15% of the cells in the VNO. The expression of this gene family is restricted to neurons within the VNO and is not observed in sensory neurons of the MOE nor in other non-neuronal cells. This array of properties is consistent with those predicted for the mammalian pheromone receptors. Proof that these sequences indeed encode pheromone receptors will require the demonstration that these receptor proteins bind pheromones and are able to transduce pheromone binding into alterations in membrane potential.

The experimental approach employed to isolate this gene family, differential screening of a cDNA library constructed from a single neuron, may be more broadly applicable to the analysis of the specific gene expression in diverse populations of cells. In the nervous system, for example, functionally distinct neurons each expressing different genes, and each projecting to different targets, are often interspersed. It has therefore been difficult to isolate RNA species unique to functionally distinct subsets of neurons within a heterogeneous cell population. The ability to generate cDNA libraries from individual cells within a diverse population of neurons may permit the identification of that subset of genes which afford a cell a unique identity.

### How Large is the Gene Family?

The number of receptor genes expressed in the two distinct olfactory organs of mammals is likely to reflect the repertoire of odors recognized by the two populations of olfactory-sensory neurons. The main olfactory organ can recognize a universe of odors which define an organism's environment, whereas the VNO largely recognizes molecules distinctive to the species that define the reproductive and social status of individuals within any given species. Olfactory receptors of the MOE is encoded by a family of about 1000 genes (Buck and Axel, 1991; Parmentier et al., 1992; Ben Arie et al., 1994). Since the range of molecules detected by the VNO is thought to be far smaller than the odors detected by the MOE, applicants anticipated that the repertoire of pheromone receptors would be far smaller as well. Gene cloning and Southern blotting with genomic DNA provide an estimate of the size of the pheromone receptor repertoire. A screen of genomic libraries with a mix of probes detect approximately 35 positive clones per genome. This value is in accord with the results of genomic blot hybridization at low stringency which identifies about 30 discrete genes with the available probes. This minimum estimate of 30-35 genes clearly provides a lower limit of the size of the VNO receptor repertoire since it is likely that the seven genes applicants have cloned do not allow us to detect all the members of the pheromone receptor gene family.

*In situ* hybridization experiments with individual probes provide an independent estimate of the number of receptor genes expressed in the VNO. Each of the seven putative pheromone receptor genes labels about 1-4% of the vomeronasal sensory neurons, whereas a mix of the genes representing the six subfamilies detects about 15% of the VNO neurons. These data suggest that a given neuron expresses only one pheromone receptor gene. Since the six subfamily probes detect about 20 genes in the chromosome at high stringency, and label 15% of the VNO neurons, applicants estimate that the repertoire of pheromone receptors may consist of about 100 distinct genes.

### The Relationship Between the Two Olfactory Organs

The sequences of the odorant receptors of the MOE and the pheromone receptors of the VNO share no apparent homology, indicating that the two olfactory sensory systems of mammals have evolved independently. This suggestion is in accord with the observation that the signal transduction machinery of the MOE cannot be detected in the neurons of the VNO. What is the evolutionary origin of the VNO? Pheromone-responsive neurons and neurons responsive to the more general class of odorants are likely to have been present throughout vertebrate evolution. With the emergence of terrestrial forms, segregation of the two types of neurons may have occurred, generating a distinct vomeronasal organ that facilitates the access and binding of the two classes of odorous ligand. Thus, terrestrial vertebrates from amphibians to mammals, including humans, retain two distinct olfactory systems, the VNO and the MOE (Bertmar, 1981; Eisthen, 1992; Potiquet, 1891; Stensaas et al., 1991; Moran et al., 1991; Garcia-Velasco and Mondragon, 1991) .

These two functional classes of sensory neurons are also apparent in invertebrate olfactory systems. These observations immediately pose the question as to whether homologs of the two different families of vertebrate . olfactory receptors are present within the genome of invertebrates. Attempts to identify genes related to the large family of MOE receptors in *C. Elegans* (Bargmann, personal communication) and *Drosophila* (Amrein, H., Vosshall, L., and Axel, R., unpublished studies; Carlson, J., personal communication) have thus far been unsuccessful. Several large families of seven transmembrane receptor genes expressed in subsets of *C*. *elegans* chemosensory neurons have recently been identified (Troemel et al., 1995) However, these sequences share no homology with the mammalian receptor sequences from either the VNO or MOE. It is possible that the identification of additional families of receptors will reveal a common evolutionary ancestor to the vertebrate and invertebrate olfactory systems. Alternatively, the differences in the chemical nature of the odorants and differences in the physiological consequences of odor recognition might suggest independent origins for the invertebrate and vertebrate olfactory system.

### The Logic of Olfactory Coding in the MOE and VNO

Analysis of the patterns of expression of receptor genes in the main olfactory system has provided significant insight into mechanisms for the diversity and specificity of odor recognition in mammals. Similarly, the isolation of the pheromone receptors from the vomeronasal organ is likely to help to elucidate the logic of olfactory perception in the vomeronasal system. The initial step in olfactory discrimination by the MOE requires the interaction of odorous ligands with one of the multiple seven transmembrane domain receptors on olfactory sensory neurons. Discrimination among odorants requires that the brain determine which of numerous receptors has been activated. Since individual olfactory sensory neurons in the MOE are likely to express only a single receptor gene, the problem of distinguishing which receptors have been activated reduces to a problem of distinguishing which neurons have been activated.

Recent experiments demonstrate that neurons expressing a given receptor, and therefore responsive to a given odorant, project their axons to one or a small number of discrete loci or glomeruli within the olfactory bulb (Vassar et al., 1994; Ressler et al. 1994; Mombaerts et al., unpublished). The positions of specific glomeruli are topographically fixed, and are conserved in the brains of all animals within a species. These data provide physical evidence that the olfactory bulb provides a two-dimensional map that identifies which of the numerous receptors have been activated within the sensory epithelium. Such a model is in accord with previous experiments demonstrating that different odors elicit spatially defined patterns of glomerular activity in the olfactory bulb (Kauer et al., 1987; Stewart et al., 1979; Lancet et al., 1982; Mori et al., 1992; Imamura et al., 1992; Katoh et al., 1993). Thus, the quality of an olfactory stimulus would therefore be encoded by the specific combination of glomeruli activated by a given odorant.

At one level, the vomeronasal system shares anatomic and physiologic features with the main olfactory system, suggesting that similar experiments with pheromone receptors might also provide insight as to how the recognition of odors by the VNO leads to the elaboration of innate behaviors. Primary olfactory sensory neurons within the vomeronasal organ project a single unbranched axon which then synapses with dendrites of mitral cells in the accessory olfactory bulb, the first relay station for vomeronasal signalling in the brain. At a molecular level, applicants have identified a family of pheromone receptor genes that encode seven transmembrane domain proteins. Individual VNO neurons are likely to express only a single receptor gene. Cells expressing a specific receptor are randomly dispersed within the apical zone of the sensory epithelium. Thus, the pattern of pheromone receptor expression shares striking similarities with the expression of odorant receptors in the MOE.

At first glance, the anatomy and molecular organization of the VNO and MOE as well as that of the main and accessory olfactory bulb appear quite similar. There are, however, important differences. In the MOE, the mitral cells, the major output neurons of the olfactory bulb, project a primary dendrite to a single glomerulus suggesting a one-to-one correspondence between mitral cell and sensory axon, such that a given mitral cell can respond to the activation of only a single class of sensory neurons. The task of discerning which sensory neurons have been activated must therefore be accomplished by integration at higher cortical centers. Mitral cells of the accessory bulb, however, exhibit a more complex primary dendritic array allowing synapse formation with more than one glomerulus and therefore more than one class of sensory neurons (Macrides et al., 1985; Takami and Graziadei, 1991). These observations suggest that in the vomeronasal system, integration permitting the detection of a specific combination of different receptors activated by pheromones may occur in the accessory olfactory bulb.

The VNO and the main olfactory system reveal striking differences in the secondary projections to the cortex and in the responses elicited by the two sensory systems. VNO neurons project directly to the amygdala and hypothalamus leading to innate and stereotypic behavioral responses (Broadwell, 1975; Scalia and Winans, 1975; Winans and Scalia, 1970; Kevetter and Winans, 1981; Krettek and Price, 1977; 1978a). In contrast, the projections from the main olfactory organ activate higher cortical centers resulting in a measured emotional or cognitive response. The projections from the vomeronasal system to the hypothalamus also control the release of luteinizing hormone release hormone (LHRH) and prolactin release hormone (PRF), increasing LH and prolactin levels both centrally and peripherally (reviewed in Keverne, 1983, Meredith and Fernandez-Fewell, 1994). In this manner, stimulation of the vomeronasal system can coordinate the activation of central neural pathways with dramatic neuroendocrine changes to elicit a characteristic array of innate reproductive and social behaviors.

The coding of olfactory information is likely to be far simpler in the vomeronasal system than in the main olfactory pathway. The receptor repertoire in the VNO is an order of magnitude smaller than in the MOE. Moreover, integration in the vomeronasal pathway is apparent in the accessory bulb and the secondary projections synapse on small number of loci in the amygdala. This is in sharp contrast to the complexity of higher cortical pathways required for processing olfactory information from the MOE. Thus, the vomeronasal system may permit the analysis of the molecular events which translate the bindings of pheromones into innate stereotypic behaviors.

### Pheromone Receptors in Humans

Until recently, the VNO in humans was thought to be an atretic organ of vestigial function. Recent reports, however, identify a structurally intact vomeronasal organ in virtually all biopsy specimens examined (Moran, et al., 1991; Stensaas, et al., 1991, Garcia-Velasco and Mondragon 1991) Activation of neurons has been observed in the human VNO in response to purified components from skin extracts (Monti-Bloch, et al, 1994), but the physiological or behavioral consequences of VNO activation remain elusive. Moreover, it has been difficult to identify human pheromones that elicit innate behavioral arrays since behavior in humans is far more likely to be tempered by learning and experience.

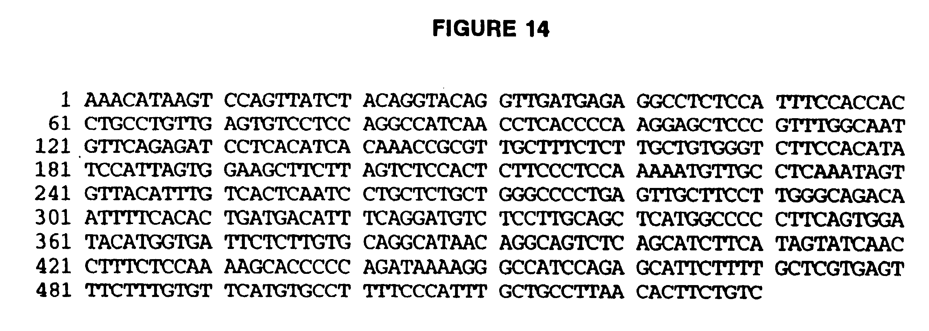

In preliminary experiments, applicants have identified homologs of the rodent VNO receptors in human genomic DNA. Low stringency screens of a human genomic library with a mix of rat VNO receptor cDNAs identifies human homologs at a frequency of about 15 per haploid genome. Partial sequence of two clones reveals 41% and 48% identity with the closest rat homologs (Figure 4C; Sequence ID. No.:15; The human amino acid sequence is designated as HG25). The nucleic acid sequence of a clone, hg25X, is presented in Figure 14 with Sequence ID. No.:7. Even though both genomic clones reveal stop codons within the coding region indicating that these two human sequences are pseudogenes, these clones provide useful tools to obtain clones which code for a functional VNO receptors. The identification of putative pheromone receptors may provide insight into the chemical nature of the pheromones, the mechanisms by which the perception of pheromones lead to innate behaviors and the possible role of this sensory system in humans.

### Experimental Procedures

### Preparation and Screening of Single Cell cDNA Libraries

The main olfactory epithelium (MOE) and vomeronasal organs (VNO) were dissected from adult Sprague-Dawley rats. The synthesis and amplification of single cell cDNA was performed according to Brady et al., 1990 with modifications. Small pieces of tissue were dissociated for 10'at 37°C in phosphate buffered saline (PBS) (without Ca²⁺, Mg²⁺) 0.025% trypsin, 0.75mM EDTA. After gentle trituration of the tissues in Dulbecco's modified Eagle's medium plus 10% calf serum, cells were collected by centrifugation and resuspended in ice cold PBS. The cell suspension was observed on a Leitz inverted microscope and olfactory sensory neurons were identified as bipolar neurons with an axonal process and a dendrite terminating in an olfactory knob. Isolated neurons were picked with a Leitz micromanipulator fitted with a pulled and beveled microcapillary. Single cells were seeded in thin-walled PCR reaction tubes (Perkin Elmer) containing 4µl of ice cold cell lysis buffer (50mM Tris-HCl (pH8.3), 75mM KCl, 3mM MgCl₂, 0.5% NP-40, containing 80ng/ml pd(T)19-24 (Pharmacia), 5u/ml Prime Rnase Inhibitor (5'-3' Inc.), 324u/ml RNAguard (Pharmacia) and 10µM each of dATP, dCTP, dGTP and dTTP). Lysis was subsequently performed for 1'at 65°C. First strand cDNA synthesis was then initiated by adding 50u Moloney murine leukemia virus- and 0.5u avian-reverse transcriptases (BRL) followed by incubation for 10' at 37°. Samples were heat-inactivated for 10' at 65° C and a poly (A) was added to the first strand cDNA product by adding an equal volume of 200mM potassium cacodylate pH7.2, 4mm CoCl2, 0.4mM DTT, 200µM dATP containing 10 units terminal transferase (Boehringer) for 15' at 37° C. Samples were heat-inactivated 10' at 65°C and the contents of each tube was brought to 100µl with a solution made of 10mM TrisHCl (pH8.3), 50mM KCl, 2.5mM MgCl2, 100µg/ml bovine serum albumin, 0.05% Triton X-100 and containing 1mM of dATP, dCTP, dGTP, dTTP, 10 units Taq polymerase (Perkin Elmer) and 5µg of the PCR primer AL1. The AL1 sequence is ATT GGA TCC AGG CCG CTC TGG ACA AAA TAT GAA TTC (T)24. PCR amplification was then performed according to the following schedule : 94°C for 1', 42°C for 2' and 72°C for 6' with 10" extension per cycle for 25 cycles. Five additional units of Tag polymerase were then added before performing 25 more cycles. In this manner, PCR amplified cDNA was synthesized from RNA of individual neurons.

Aliquots of single cell cDNA were run on 1% agarose gels, blotted on nylon membrane (Hybond N+, Amersham) and hybridized with several DNA probes to determine the representation of specific sequences in amplified cDNA. The probes included highly expressed genes (tubulin, OMP), gene expressed at lower level (Go) as well as genes whose expression is restricted to either MOE (G_{olf}) or VNO (Gᵢ₂ₐ) neuron. The relative level of these genes in amplified cDNA prepared from individual neurons is in accord with levels determined by either *in situ* hybridization or screening more classical cDNA libraries. These data suggest that the amplified cDNA from individual neurons contains an accurate representation of sequences in mRNA.

One microgram of the cDNA prepared from VNO neuron 1 was purified by phenol/chloroform extraction, digested with EcoR1, ligated into EcoR1 predigested and dephosphorylated λZAPII phage arms (Stratagene) and packaged according to standard procedures. The library prepared from VNO neuron 1 consisted of 5 X 10⁴ pfus with an average insert size of 600bp. The frequency of OMP and tubulin positive plaques (0.2%) suggested that the representation of a given RNA was not biased during the construction of the library.

Amplified cDNA from single cells was used as probe by reamplifying 1µl of neuron cDNA for 10 cycles with the AL1 primer in the presence of 100µCi of 32P-dCTP. One thousand recombinant phages from VNO neuron 1 library were plated at low density and triplicate filters (Hybond N+, Amersham) were prehybridized at 65°C in 0.5M sodium phosphate buffer (pH7.3) containing 1% bovine serum albumin and 4% SDS. Hybridization was carried out in the same buffer and at 65° C after adding 10⁷cpm/ml of the amplified cDNA probe made from either VNO neuron 1, VNO neuron 2 or from a MOE neuron. Filters were washed three times at 65°C in 0.5% SDS and 0.5X SSC. Twenty phage plaques showing specific hybridization with the VNO neuron 1 probe were isolated. Phage inserts were amplified by PCR, run on 1% agarose gels, transferred to nylon membranes and were again hybridized with single cell cDNA probes as described above. Phages 18 and 19 contained cDNA inserts which appeared to hybridize only to VNO neuron 1 cDNA probe. Plasmids were obtained from the isolated phages by performing phagemid rescue as instructed by the manufacturer (Stratagene). DNA sequence analysis was performed on plasmid DNAs using the Sequenase system (United States Biochemical Corp).

### Isolation and Analysis of Full-length cDNA Clones

Poly A+ RNA was isolated from VNOs dissected from adult male or female rats using the polyA+ isolation kit (Stratagene) according to the manufacturers instructions. cDNA libraries were prepared in the λZapII vector (Stratagene) according to standard procedures (Sambrook et al., 1989). 2 X 10⁵ independent recombinant phages from the male and female VNO cDNA libraries were screened under high stringency hybridization ( 68∞C in 0.5M sodium phosphate buffer (pH7.3) containing 1% bovine serum albumin and 4% SDS) with a ³²P- labelled probe (Prime-it, Stratagene) prepared from the VNO neuron 1 specific clone 18. This allowed the isolation of two full length cDNA clones, VN1 and VN2. In further screens one additional crosshybridizing cDNA clone, VN3, was obtained by low stringency hybridization (55∞C in the same buffer as described above) of a mix of VN1 and VN2 probes to the VNO cDNA libraries. Conserved motifs within these cDNA clones were used to generate PCR primers which were then used to amplify additional sequences from the VNO cDNA libraries. This PCR product, along with the three cDNA clones were used as probes in further hybridizations to obtain four additional full-length cDNAs.

### Southern Blotting and In Situ Hybridization Analysis

Genomic DNA prepared from Sprague-Dawley rat liver was digested with the restriction enzymes EcoR1, Pst1 or Hind3, size fractionated on 0.8% agarose gels, and blotted into nylon membrane (Sambrook et al., 1989). The membranes were cross-linked under UV light, prehybridized and hybridized in 0.5M sodium phosphate buffer (pH7.3) containing 1% bovine serum albumin and 4% SDS at either high (68° C) or low (55° C) stringency conditions. Lambda fix II genomic libraries made from human placenta (Stratagene) and Sprague Dawley rats were screened under low stringency conditions.

In situ hybridization was performed as described (Schaeren-Wiemers and Gerfin-Moser, 1993) using full-length clones VN1 to VN7 as templates to synthesize digoxygenin-labeled cRNA probes. Sequences corresponding to the BamH1-Asp718 fragment of OMP cDNA (Rogers et al., 1987) were used to synthesize a 1kb OMP probe. The sequence encompassing the transmembrane domains 3 through 7 of MOE receptor M12 was isolated by PCR.

### References

Bakalyar, H.A., and Reed, R.R. (1990). Identification of a specialized adenylyl cyclase that may mediate odorant detection. Science *250*, 1403-1406.
Baldwin, J.M. (1993). The probable arrangement of the helices in G protein-coupled receptors. The EMBO Journal *12*(4), 1693-1703.
Bean, N.J. (1982). Modulation of agonistic behavior by the dual olfactory system in male mice. Physiol. Behav. *29*, 433-437.
Ben Arie, N., Lancet, D., Taylor, C., Khen, M., Walker, N., Ledbetter, D.H., Carrozzo, R., Patel, K., Sheer, D., Lehrach, H., and North, M.A. (1994). Olfactory receptor gene cluster on human chromosome 17: possible duplication of an ancestral receptor repertoire. Hum. Mol. Gen. *3,* 229-235.
Bertmar, G. (1981). Evolution of vomeronasal organs in vertebrates. Evolution *35,* 359-366.
Brady, G., Barbara, M., and Iscove, N.N. (1990). Representative *in vitro* cDNA amplification from individual hemopoietic cells and colonies. Methods in Mol. Cell. Biol. *2,* 17-25.
Broadwell, R.D. (1975). Olfactory relationships of the Telencephalon and Diencephalon in the rabbit. I. An autoradiographic study of the efferent connections of the main and accessory olfactory bulbs J. Comp. Neural. *163,* 329-346.
Buck, L., and Axel, R. (1991). A novel multigene family may encode odorant receptors: a molecular basis for odor recognition. Cell *65,* 175-187.
Halpern, M., Shapiro, L.S., and Jia, C. (1995). Differential localization of G proteins in the opossum vomeronasal system. Brain Research *677*, 157-161.
Chess, A., Simon, I., Cedar, H., and Axel, R. (1994). Allelic inactivation regulates olfactory receptor gene expression. Cell *78*, 823-834.
Clancy, A.N., Coquelin, A., Macrides, F., Gorski, R.A., and Nobel, E.P. (1984). Sexual behavior and aggression in male mice: involvement of the vomeronasal system. J. Neurosci. *4,* 2222-2229.
Dhallan, R.S., Yau, K.W,, Schrader, K.A., and Reed, R.R. (1990). Primary structure and functional expression of a cyclic nucleotide-activated channel from olfactory neurons. Nature *347*, 184-187.
Eisthen, H.L. (1992). Phylogeny of the vomeronasal system and of receptor cell types in the olfactory and vomeronasal epithelia of vertebrates. Micro. Res. Tech. *23*, 1-21.
Farbman, A.I. (1992). Cell biology of olfaction. In Developmental and Cell Biology Series (Cambridge University Press , Canada).
Garcia-Velasco, J. and Mondragon M. (1991). The incidence of the vomeronasal organ in 1000 human subjects and its possible clinical significance. J. Steroid Biochem. Molec. Biol. *39*(4B), 561-564.
Halpern, M. (-1987). The Organization and Function of the Vomeronasal System. Ann. Rev. Neurosci. *10,* 325-362.
Henzel, W.J., Rodriguez, H., Singer, A.G., Stults, J.T., Macrides, F., Agosta, W.C., and Niall, H. (1988). The primary structure of aphrodisin. J. Biol. Chem. *263*(32), 16682-16687.
Imamura, K., Mataga, N., and Mori, K. (1992). Coding of odor molecules by mitral/tufted cells in rabbit olfactory bulb. I. Aliphatic compounds. J. Neurophysiol. *68,* 1986-2002.
Jacobson, L. (1811). Description anatomique d'un organe observe dans les Mammiferes. Ann. Mus. Hist Natn Paris *18,* 412-424.
Johns, M.A., Feder, H.H., Komisaruk, B.R., and Mayer, A.D. (1978). Urine-induced reflex ovulation in anovulatory rats may be a vomeronasal effect. Nature *272*, 446-448.
Jones, D.T., and Reed, R.R. (1989). Golf: An olfactory neuron specific G-protein involved in odorant signal transduction. Science *244,* 790-795.
Katoh, K., Koshimoto, H., Tani, A., and Mori, K. (1993). Coding of odor molecules by mitral/tufted cells in rabbit olfactory bulb. II. Aromatic compounds. J. Neurophysiol. *70,* 2161-2175.
Kauer, J.S., Senseman, D.M., and Cohen, L.B. (1987). Odor-elicited activity monitored simultaneously from 124 regions of the salamander olfactory bulb using a voltage-senstive dye. Brain Res. *418,* 255-261.
Keverne, E.- B. (1983) Pheromonal influences on the endocrine regulation of reproduction. Trends Neurosc. *6* 381-384.
Kevetter, G.A., and Winans, S.S. (1981). Connections of the corticomedial amygdala in the golden hamster. I. Efferents of the "Vomeronasal Amygdala." J. Comp. Neurol. *197*, 81-98.
Krettek, J.E., and Price, J.L. (1977). Projections from the amygdaloid complex to the cerebral cortex and thalamus in the rat and cat. J. Comp. Neurol. *172,* 687-722.
Krettek, J.E., and Price, J.L. (1978). Amygdaloid projections to subcortical structures within the basal forebrain and brainstem in the rat and cat. J. Comp. Neural. *178,* 225-254.
Lancet, D., Greer, C.A., Kauer, J.S., and Shepherd, G.M. (1982). Mapping of odor-related neuronal activity in the olfactory bulb by high resolution 2-deoxyglucose autoradiography. Proc. Natl. Acad. Sci. USA *79*, 670-674.
Lomas, D.E., and Keverne, E.B. (1982). Role of the vomeronasal organ and prolactin in the acceleration of puberty in female mice. J. Reprod. Fert. *66,* 101-107.
Ludwig, J., Marglit, T., Eismann, E., Lancet, D., and Kaupp, U.B. (1990). Primary structure of a cAMP-gated channel from bovine olfactory epithelium. FEBS Lett. *270*, 24-29.
Macrides, F., Schoenfeld, T.A., Marchand, J.E., and Clancy, A. N. (1985). Evidence for morphologically, neurochemically and functionally heterogeneous classes of mitral and tufted cells in the olfactory bulb. Chemical Senses *10*(2), 175-202.
Melrose, D.R., Reed, H.C., and Patterson, R.L. (1971). Androgen steroids associated with boar odour as an aid to the detection of oestrus in pig artificial insemination. Brit. Vet. J. *127*(10), 497-502.
Meredith, M. (1986). Vomeronasal organ removal before sexual experience impairs male hamster mating behavior. Physiol. and Behavior *36*, 737-743.
Meredith, M., and Fernandez-Fewell, G. (1994). Vomeronasal system, LHRH, and sex behaviour. Psychoneuroendocrinology. *19* (5-7), 657-672.
Michael, R.P., and Keverne, E.B. (1968). Pheromones in the communication of sexual status in primates. Nature *218*(143), 746-749.
Monti-Bloch L., Jennings-White, C., Dolberg, D. S., and Berliner, D. L. (1994). The human vomeronasal system. Psychoneuroendocrinology. *19* (5-7), 673-686.
Moran, D.T, Jafek, B.W., and Rowley III, J. C. (1991). The vomeronasal (Jacobson's) organ in man: ultrastructure and frequency of occurrence. J. Steroid Biochem. Molec. Biol. *39*(4B), 545-552.
Mori, K., Mataga, N., and Imamura, K. (1992). Differential specificities of single mitral cells in rabbit olfactory bulb for a homologous series of fatty acid odor molecules. J. Neurophysiol. *67*, 786-789.
Ngai, J., Chess, A., Dowling, M.M., Necles, N., Macagno, E.R., and Axel R. (1993a). Coding of olfactory information: topography of odorant receptor expression in the catfish olfactory epithelium. Cell *72,* 667-680.
Parmentier, M., Libert, F., Schurmans, S., Schiffmann, S., Lefort, A., Eggerickx, D. Ledent, C., Mollereau. C., Gerard, C., Perret, J., Grootgoed, A., and Vassart, G. (1992). Expression of members of the putative olfactory receptor gene family in mammalian germ cells. Nature *355*, 453-455.
Potiquet, M. (1891). Le canal de Jacobson. Rev. Laryng. (Paris) *2*,737-753.
Probst, W.C., Snyder, L.A., Schuster, D.I., Brosius, J., and Sealfon, S.C. (1992). Sequence Alignment of the G-Protein Coupled Receptor Superfamily. In DNA and Cell Biology, Vol. II(1) (Mary Liebert, Inc. Publishers, NY), pp. 1-20.
Ressler, K.J., Sullivan, S.L., and Buck, L.B. (1993). A zonal organization of odorant receptor gene expression in the olfactory epithelium. Cell, *73*, 597-609.
Ressler, K.J., Sullivan, S., and Buck, L.B. (1994). Information coding in the olfactory system: Evidence for a stereotyped and highly organized epitope map in the olfactory bulb. Cell *79*, 1245-1255.
Reynolds, J., and Keverne, E.B. (1979). The accessory olfactory system and its role in the pheromonally mediated suppression of oestrus in grouped mice. J. Reprod. Pert. *57,* 31-35.
Rogers, R.E., Dasgupta, P., Gubler, U., Grillo, M., Khew-Goodall, Y., and Margolis, F.L. (1987). Moleculr cloning and sequencing of a cDNA for olfactory marker protein. Proc. Natl. Acad. Sci. USA *84,* 1704-1708.
Sambrook, J., Fritsch, E.F., and Maniatis, T. (1989). Molecular Cloning: A Laboratory Manual, Second Edition (Cold Spring Harbor, New York: Cold Spring Harbor Laboratory Press).
Scalia, F., and Winans, S.S. (1975). The differential projections of the olfactory bulb and accessory olfactory bulb in mammals. J. Comp. Neur. *161,* 31-56.
Schaeren-Wiemers, N., and Gerlin-Moser, A. (1993). A single protocol to detect transcripts of various types and expression levels in neural tissue and cultures cells: *in situ* hybridization using digoxigenin-labeled cRNA probes. Histochemistry *100,* 431-440.
Singer, A.G. (1991). A chemistry of mammalian pheromones. J. Steroid Biochem. Molec. Biol. *39*(4B), 627-632.
Sorensen, P.W., Hara, T.J., Stacey, N.E., and Goetz, F.W. (1988). F prostaglandins function as potent olfactory stimulants that comprise the postovulatory female sex pheromone in goldfish. Biol. of Reproduction *39,* 1039-1050.
Stacey, N.E., and Sorensen, P.W. (1986). 17a, 20b-dihydroxy-4-pregnen-3-one: a steroidal primer pheromone increasing milt volume in the goldfish, *Carassius auratus.* Can. J. Zool. *64,* 2412-2417.
Stensaas, L.J., Lavker, R.M., Monti-Bloch, L., Grossier, B.I., and Berliner, D.L. (1991). Ultrastructure of the human vomeronasal organ. J. Steroid Biochem. Molec. Biol. *39*(4B), 553-560.
Stewart, W.B., Kauer, J.S., and Shepherd, G.M. (1979). Functional organization of rat olfactory bulb analyzed by the 2-deoxyglucose method. J. Comp. Neurol. *185*, 715-734.
Strader, C.D., Fong, T.M., Tota, M.R., and Underwood, D. (1994). Structure and function of G protein-coupled receptors. Annu. Rev. Biochem. *63,* 101-132.
Takami, S., and Graziadie, P.P. (1991). Light microscopic Golgi study of mitral/tufted cells in the accessory olfactory bulb of the adult rat. J. Comp. Neurol. *311*, 65-83.
Troemel, E., Chou, J., Dwyer, N.D., Colbert, H.A., and Bargmann, C.I. (1995). Divergent seven transmembrane receptors are candidate chemosensory receptors in C. elegans. Cell, in press.
Vassar, R., Ngai, J., and Axel, R. (1993). Spatial segregation of odorant receptor expression in the mammalian olfactory epithelium. Cell *74,* 309-318.
Vassar, R., Chao, S.K., Sitcheran, R., Nunez, J..M., Vosshall, L.B., and Axel, R. (1994). Topographic organization of sensory projections to the olfactory bulb. Cell *79,* 981-991.
Winans, S.S., and Scalia, F. (1970). Amygdaloid nucleus: New afferent input from the vomeronasal organ. Science *170*, 325-344.
Wysocki, C.J. (1989). Vomeronasal chemoreception: It's role in reproductive fitness and physiology. In Neural Control of Reproductive Function (Alan R. Liss, Inc.) pp. 545-566.
Wysocki, C.J., and Lepri. J.J. (1991). Consequences of removing the vomeronasal organ. J. Steroid Biochem. Molec. Biol. *39*, 661-669.

## Claims

1. A nucleic acid molecule encoding a vertebrate pheromone receptor or a biologically active or antigenic fragment thereof selected from the group consisting of:
(a) nucleic acid molecules encoding a polypeptide having an amino acid sequence as depicted in any of SEQ ID NOs. 8 to 15 or 17 or 18;
(b) nucleic acid molecules having a nucleic acid sequence as depicted in any one of SEQ ID NOs. 1 to 7;
(c) nucleic acid molecules encoding the polypeptide having the amino acid sequence of the polypeptide encoded by the DNA inserts contained in any one of ATCC Nos. 97294 to 97299;
(d) nucleic acid molecules having the coding sequence of the DNA insert contained in any one of ATCC Nos. 97294 to 97299;
(e) nucleic acid molecules encoding a fragment, analog or derivative of a polypeptide encoded by a nucleic acid molecule of any one of (a) to (d); and
(f) nucleic acid moledules,' the complementary strand of which hybridizes with a nucleic acid molecule as defined in any one of (a) to (e).

2. The nucleic acid molecule of claim 1 which is DNA or RNA.

3. The nucleic acid molecule of claim 2 which is cDNA or genomic DNA.

4. The nucleic acid molecule of any one Of claims 1-3, wherein the nucleic acid molecule encodes a mammalian pheromone receptor.

5. The nucleic acid molecule of claim 4, wherein the nucleic acid molecule encodes a human or rat pheromone receptor.

6. A nucleic acid molecule of at least 12 nucleotides capable of specifically hybridizing with the sequence of a nucleic acid molecule of any one of claims 1 to 5.

7. The nucleic acid molecule of claim 6 which is DNA or RNA.

8. A method for identifying DNA inserts encoding pheromone receptors comprising:
(a) generating DNA libraries which contain clones carrying inserts from a sample which contains at least one vomeronasal sensory neuron;
(b) contacting clones from the cDNA libraries generated in step (a) with the nucleic acid molecule of claim 6 or 7 in appropriate conditions permitting the hybridization of the nucleic acid molecules of the clones and the nucleic acid molecule;
(c) selecting clones which hybridized with the nucleic acid molecule; and
(d) isolating the clones which carry the hybridized inserts, thereby identifying the inserts encoding the pheromone receptors.

9. The method of claim 8, after step (c), further comprising:
(a) amplifying the inserts from the selected clones by polymerase chain reaction;
(b) hybridizing the amplified inserts with probes from the individual vomeronasal neuron; and
(c) isolating the clones which carry the hybridized inserts, thereby identifying the inserts encoding the pheromone receptors.

10. The method of claims 8 or 9, wherein the probes are cDNA probes.

11. The method of claim 8, wherein the sample contains only one individual vomeronasal sensory neuron.

12. The method of claim 8 or 11, wherein the libraries are cDNA libraries or genomic libraries.

13. A vector which comprises the nucleic acid molecule of any one of claims 1-5.

14. The vector of claim 13, wherein the nucleic acid molecule is operatively linked to a regulatory element.

15. The vector of claim 13 or 14 which is a plasmid.

16. The plasmid of claim 15 designated VN1 (ATCC Accession No. 97294), VN3 (ATCC Accession No. 97295), VN4 (ATCC Accession No. 97296), VN5 (ATCC Accession No. 97297), VN6 (ATCC Accession No. 97298) or VN7 (ATCC Accession No. 97299).

17. A method of transforming cells which comprises transfecting a host cell with a suitable vector of any one of the claims 13 to 16.

18. A host vector system for the production of a polypeptide having the biological activity of a vertebrate pheromone receptor which comprises the vector of any one of claims 13 to 16 and a suitable host.

19. The host vector system of claim 18, wherein the suitable host is a bacterial cell, yeast cell, insect cell or animal cell.

20. A method for producing a polypeptide having the biological activity of a vertebrate pheromone receptor which comprises growing the host vector system of claim 18 or 19 under conditions permitting production of the polypeptide and recovering the polypeptide produced.

21. A polypeptide encoded by the nucleic acid molecule of any one of claims 1 to 5.

22. An antibody capable of specifically binding to the polypeptide of claim 21.

23. An antibody capable of competitively inhibiting the binding of the antibody of claim 22.

24. The antibody of claim 22 or 23 which is a monoclonal antibody.

25. A method for identifying a compound capable of specifically binding to a vertebrate pheromone receptor which comprises
(a) contacting the polypeptide of claim 21 with an appropriate amount of the compound under conditions permitting binding of the compound to the polypeptide; and
(b) detecting the presence of the compound specifically bound to the polypeptide, thereby determining whether the compound specifically binds to the polypeptide.

26. A transgenic non-human living organism expressing the nucleic acid molecule of claim 1.

27. The transgenic non-human living organism of claim 26 which is a transgenic animal.

## Patentansprüche

1. Nucleinsäuremoleküi, das einen Vertebraten-Pheromon-Rezeptor oder ein biologisch aktives oder antigenes Fragment davon codiert, ausgewählt aus der Gruppe bestehend aus:
(a) Nucleinsäuremolekülen, die ein Polypeptid mit einer Aminosäuresequenz wie in einer der SEQ ID Nummern 8 bis 15 oder 17 oder 18 dargestellt codieren;
(b) Nucleinsäuremolekülen, mit einer Nucleinsäuresequenz wie in einer der SEQ ID Nummern 1 bis 7 dargestellt;
(c) Nucleinsäuremolekülen, die das Polypeptid codieren, weiches die Aminosäuresequenz des Polypeptids hat, das durch die DNA-Insertionen codiert wird, die in einer der ATCC NR. 97294 bis 97299 enthalten sind;
(d) Nucteinsäuremolekülen, welche die codierende Sequenz der DNA-Insertion haben, die in einer der ATCC NR. 97294 bis 97299 enthalten ist;
(e) Nucleinsäuremolekülen, die ein Fragment, Analog oder Derivat eines Polypeptids codieren, welches durch ein Nucleinsäuremolekül nach einem der Punkte (a) bis (d) codiert wird; und
(f) Nucleinsäuremolekülen, deren komplementärer Strang mit einem Nucleinsäuremolekül, wie es in einem der Punkte (a) bis (e) definiert ist, hybridisiert.

2. Nucleinsäuremolekül nach Anspruch 1, das DNA oder RNA ist.

3. Nucleinsäuremolekül nach Anspruch 2, das cDNA oder genomische DNA ist.

4. Nucleinsäuremolekül nach einem der Ansprüche 1 bis 3, wobei das Nucleinsäuremolekül einen Säuger-Pheromon-Rezeptor codiert.

5. Nucleinsäuremolekül nach Anspruch 4, wobei das Nucleinsäuremolekül einen menschlichen oder einen Ratten-Pheromon-Rezeptor codiert.

6. Nucleinsäuremolekül mit mindestens 12 Nukleotiden, die spezifisch mit der Sequenz eines Nucleinsäuremoleküls nach einem der Ansprüche 1 bis 5 hybridisieren können.

7. Nucleinsäuremolekül nach Anspruch 6, das DNA oder RNA ist.

8. Verfahren zur Identifizierung von Pheromon-Rezeptoren codierenden DNA-Insertionen, das umfasst:
(a) Generierung von DNA-Banken, die Clone enthalten, welche Insertionen aus einer Probe enthalten, die mindestens ein vomeronasalsensorisches Neuron enthält;
(b) Inkontaktbringen der Clone aus den cDNA-Banken, die in Schritt (a) generiert wurden, mit dem Nucleinsäuremolekül nach Anspruch 6 oder 7 unter geeigneten Bedingungen, welche die Hybridisierung von Nucleinsäuremolekülen der Clone und dem Nucleinsäuremolekül zulassen.;
(c) Selektion der Clone, die mit dem Nucleinsäuremolekül hybridisierten; und
(e) Isolierung der Clone, welche die hybridisierten Insertionen tragen, und dadurch Identifizieren der Insertionen, die Pheromon-Rezeptoren codieren.

9. Verfahren nach Anspruch 8, das nach Schritt (c) weiterhin umfasst:
(a) Amplifikation der Insertionen der selektierten Clone durch Polymerasekettenreaktion;
(b) Hybridisierung der amplifizierten Insertionen mit Sonden des einzelnen vomeronasalen Neurons; und
(c) Isolierung der Clone, welche die hybridisierten Insertionen tragen, und dadurch identifizieren der Insertionen, die Pheromon-Rezeptoren codieren.

10. Verfahren nach Anspruch 8 oder 9, wobei die Sonden cDNA-Sonden sind.

11. Verfahren nach Anspruch 8, wobei die Probe nur ein einzelnes vomeronasales sensorisches Neuron enthält.

12. Verfahren nach Anspruch 8 oder 11, wobei die Banken cDNA- oder genomische Banken sind.

13. Vektor, der das Nucleinsäuremolekül nach einem der Ansprüche 1 bis 5 umfasst.

14. Vektor nach Anspruch 13, wobei das Nucleinsäuremolekül mit einem regulatorischen Element funktionell verknüpft ist.

15. Vektor nach Anspruch 13 oder 14, der ein Plasmid ist.

16. Plasmid nach Anspruch 15, das als VN1 (ATCC-Hinterlegungs-NR. 97294), VN3 (ATCC-Hinterlegungs-NR. 97295), VN4 (ATCC-Hinterlegungs-NR. 97296), VN5 (ATCC-Hinterlegungs-NR. 97297), VN6 (ATCC-Hinterlegungs-NR. 97298) oder VN7 (ATCC-Hinterlegungs-NR. 97299) bezeichnet ist.

17. Verfahren zur Transformation von Zellen, welches die Transfektion einer Wirtszelle mit einem geeigneten Vektor nach einem der Ansprüche 13 bis 16 umfasst.

18. Wirt-Vektor-System zur Herstellung eines Polypeptids mit der biologischen Aktivität eines Vertebraten-Pheromon-Rezeptors, welches den Vektor nach einem der Ansprüche 13 bis 16 und einen geeigneten Wirt umfasst.

19. Wirt-Vektor-System nach Anspruch 18, wobei der geeignete Wirt eine Bakterienzelle, Hefezelle, lnsektenzelle oder tierische Zelle ist.

20. Verfahren zur Herstellung eines Polypeptids mit der biologischen Aktivität eines Vertebraten-Pheromon-Rezeptors, umfassend das Wachstum des Wirt-Vektor-Systems nach Anspruch 18 oder 19 unter Bedingungen, welche die Herstellung des Polypeptids und die Gewinnung des hergestellten Polypeptids zulassen.

21. Polypeptid, welches durch das Nucleinsäuremolekül nach einem der Ansprüche 1 bis 5 codiert wird.

22. Antikörper, der spezifisch an das Polypeptid nach Anspruch 21 binden kann.

23. Antikörper, der die Bindung des Antikörpers nach Anspruch 22 kompetitiv hemmen kann.

24. Antikörper nach Anspruch 22 oder 23, der ein monoclonaler Antikörper ist.

25. Verfahren zur Identifizierung einer Verbindung, die spezifisch an einen Vertebraten-Pheromon-Rezeptor binden kann, welches umfasst:
(a) Inkontaktbringen des Polypeptids nach Anspruch 21 mit einer geeigneten Menge der Verbindung unter Bedingungen, die eine Bindung der Verbindung an das Polypeptid zulassen; und
(b) Nachweis der Anwesenheit der Verbindung, die spezifisch an das Polypeptid gebundenen ist, und dabei Bestimmen, ob die Verbindung spezifisch an das Polypeptid bindet.

26. Transgener, nicht-menschlicher lebender Organismus, der das Nucleinsäuremolekül nach Anspruch 1 exprimiert.

27. Transgener, nicht-menschlicher lebender Organismus nach Anspruch 26, der ein transgenes Tier ist.

## Revendications

1. Molécule d'acide nucléique codant pour un récepteur de phéromone de vertébré ou un fragment biologiquement actif ou antigénique de celui-ci choisi dans le groupe constitué de :
(a) molécules d'acide nucléique codant pour un polypeptide ayant une séquence d'acides aminés comme décrite dans une quelconque des SEQ ID N° 8 à 15 ou 17 ou 18 ;
(b) molécules d'acide nucléique ayant une séquence d'acide nucléique comme décrite dans une quelconque des SEQ ID N° 1 à 7 ;
(c) molécules d'acide nucléique codant pour un polypeptide ayant la séquence d'acides aminés du polypeptide codé par les inserts d'ADN contenus dans un quelconque des ATCC N° 97294 à 97299 ;
(d) molécules d'acide nucléique ayant la séquence codante de l'insert d'ADN contenu dans un quelconque des ATCC N° 97294 à 97299 ;
(e) molécules d'acide nucléique codant pour un fragment, un analogue ou un dérivé d'un polypeptide codé par une molécule d'acide nucléique selon l'une quelconque des étapes (a) à (d) ;et
(f) molécules d'acide nucléique dont le brin complémentaire s'hybride avec une molécule d'acide nucléique comme définie dans l'une quelconque des étapes (a) à (e).

2. Molécule d'acide nucléique selon la revendication 1 qui est un ADN ou un ARN.

3. Molécule d'acide nucléique selon la revendication 2 qui est un ADNc ou un ADN génomique.

4. Molécule d'acide nucléique selon l'une quelconque des revendications 1 à 3, dans laquelle la molécule d'acide nucléique code pour un récepteur de phéromone de mammifère.

5. Molécule d'acide nucléique selon la revendication 4, dans laquelle la molécule d'acide nucléique code pour un récepteur de phéromone d'humain ou de rat.

6. Molécule d'acide nucléique ayant au moins 12 nucléotides capables de s'hybrider spécifiquement avec la séquence d'une molécule d'acide nucléique selon l'une quelconque des revendications 1 à 5.

7. Molécule d'acide nucléique selon la revendication 6 qui , est un ADN ou un ARN.

8. Procédé destiné à identifier des inserts d'ADN codant pour des récepteurs de phéromone comprenant :
(a) la génération de banques d'ADN qui contiennent des clones portant des inserts provenant d'un échantillon qui contient au moins un neurone sensoriel voméronasal ;
(b) la mise en contact des clones des banques d'ADNc générées dans l'étape (a) avec la molécule d'acide nucléique de la revendication 6 ou 7 dans des conditions appropriées permettant l'hybridation des molécules d'acide nucléique des clones et de la molécule d'acide nucléique ;
(c) la sélection des clones qui s'hybrident avec la molécule d'acide nucléique ;et
(d) l'isolement des clones qui portent les inserts hybridés identifiant ainsi les inserts codant pour les récepteurs de phéromone.

9. Procédé selon la revendication 8, après l'étape (c), qui comprend en outre :
(a) l'amplification des inserts des clones sélectionnés par la réaction d'amplification en chaîne par polymérase ;
(b) l'hybridation des inserts amplifiés avec des sondes provenant du neurone voméronasal individuel ;et
(c) l'isolement des clones qui portent les inserts hybridés, identifiant ainsi les inserts codant pour les récepteurs de phéromone.

10. Procédé selon les revendications 8 ou 9, dans lequel les sondes sont des sondes d'ADNc.

11. Procédé selon la revendication 8, dans lequel l'échantillon ne contient qu'un neurone sensitif voméronasal individuel.

12. Procédé selon les revendications 8 ou 11, dans lequel les banques sont des banques d'ADNc ou des banques génomiques.

13. Vecteur qui comprend la molécule d'acide nucléique selon. l'une quelconque des revendications 1 à 5.

14. Vecteur selon la revendication 13, dans lequel la molécule d'acide nucléique est liée de façon fonctionnelle à un élément de régulation.

15. Vecteur selon les revendications 13 ou 14 qui est un plasmide.

16. Plasmide selon la revendication 15 désigné par VN1 (N° d'accès ATCC 97294), VN3 (N° d'accès ATCC 97295), VN4 (N° d'accès ATCC 97296), VN5 (N° d'accès ATCC 97297), VN6 (N° d'accès ATCC 97298) ou VN7 (N° d'accès ATCC 97299).

17. Procédé de transformation des cellules qui comprend la transfection d'une cellule hôte avec un vecteur adapté selon l'une quelconque des revendications 13 à 16.

18. Système hôte-vecteur destiné à la production d'un polypeptide ayant l'activité biologique d'un récepteur de phéromone de vertébré qui comprend le vecteur selon l'une quelconque des revendications 13 à 16 et un hôte adapté.

19. Système hôte-vecteur selon la revendication 18, dans lequel l'hôte adapté est une cellule bactérienne, une cellule de levure, une cellule d'insecte ou une cellule animale.

20. Procédé destiné à produire un polypeptide ayant l'activité biologique d'un récepteur de phéromone de vertébré qui comprend la croissance du système hôte-vecteur selon la revendication 18 ou 19 dans des conditions permettant la production d'un polypeptide et la récupération du polypeptide produit.

21. Polypeptide codé par la molécule d'acide nucléique selon l'une quelconque des revendications 1 à 5.

22. Anticorps capable de se lier spécifiquement au polypeptide de la revendication 21.

23. Anticorps capable d'inhiber par compétition la liaison de l'anticorps de la revendication 22.

24. Anticorps des revendications 22 ou 23 qui est un anticorps monoclonal.

25. Procédé destiné à identifier un composé capable de se lier spécifiquement à un récepteur de phéromone de vertébré qui comprend
(a) la mise en contact du polypeptide de la revendication 21 avec une quantité appropriée du composé dans des conditions permettant la liaison du composé au polypeptide ;et
(b) la détection de la présence du composé spécifiquement lié au polypeptide, déterminant ainsi si le composé se lie spécifiquement au polypeptide.

26. Organisme vivant non humain transgénique exprimant la molécule d'acide nucléique selon la revendication 1.

27. Organisme vivant non humain transgénique selon la revendication 26 qui est un animal transgénique.


### SEQUENCE LISTING

(1) GENERAL INFORMATION:
   (i) APPLICANT: Dulac, Catherine
      Axel, Richard
   (ii) TITLE OF INVENTION: Cloning Of vertebrate Pheromone Receptors And Uses Thereof
   (iii) NUMBER OF SEQUENCES: 18
   (iv) CORRESPONDENCE ADDRESS:
      (A) ADDRESSEE: Cooper & Dunham LLP
      (B) STREET: 1185 Avenue of the Americas
      (C) CITY: New York
      (D) STATE: New York
      (E) COUNTRY: United States
      (F) ZIP: 10036
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: White, John P.
      (B) REGISTRATION NUMBER: 28678
      (C) REFERENCE/DOCKET NUMBER: 48557
   (ix) TELECOMMUNICATION INFORMATION:
      (A) TELEPHONE: 212 278 0400
      (B) TELEFAX: 212 391 0526
(2) INFORMATION FOR SEQ ID NO:1:
   (i) SEQUENCE CHARACTERISTICS:
      (A) LENGTH: 530 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:1.:
   AAACATAAGT CCAGTTATCT ACAGGTACAG GTTGATGAGA GGCCTCTCCA TTTCCACCAC    60
   CTGCCTGTTG AGTGTCCTCC AGGCCATCAA CCTCACCCCA AGGAGCTCCC GTTTGGCAAT   120
   GTTCAGAGAT CCTCACATCA CAAACCGCGT TGCTTTCTCT TGCTGTGGGT CTTCCACATA   180
   TCCATTAGTG GAAGCTTCTT AGTCTCCACT CTTCCCTCCA AAAATGTTGC CTCAAATAGT   240
   GTTACATTTG TCACTCAATC CTGCTCTGCT GGGCCCCTGA GTTGCTTCCT TGGGCAGACA   300
   ATTTTCACAC TGATGACATT TCAGGATGTC TCCTTGCAGC TCATGGCCCC CTTCAGTGGA   360
   TACATGGTGA TTCTCTTGTG CAGGCATAAC AGGCAGTCTC AGCATCTTCA TAGTATCAAC   420
   CTTTCTCCAA AAGCACCCCC AGATAAAAGG GCCATCCAGA GCATTCTTTT GCTCGTGAGT   480
   TTCTTTGTGT TCATGTGCCT TTTCCCATTT GCTGCCTTAA CACTTCTGTC              530
(2) INFORMATION FOR SEQ ID NO:2:
   (i) SEQUENCE CHARACTERISTICS:
      (A) LENGTH: 1385 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:2:
   TTTCGGCACG AGTTCACCTG CCCTCGAATT TCAATTTGAG TAAGTGACCA GCAATGGAGT 60
   ACAGAATCAG AAGATGGTTG GATCCCAGGC AGGCTGTGGG AGGAGGAACT CTGGAACTGC 120
   ATGAGGAGTT TGAGCACCTG CCATGGAGTA GCTGATCTCT GAGGACCCCT CACACAGGTC 180
   CTGTGTTCTA CATCAAGTGC ATATTTTTCC TAGGATATTC ATTTCCGTAA GTCCTGAAAT 240
   TACTTAATTT TTATAGGAGT TCTCATATAT GATGAATAAG AACAGCAGAC TCTACACTGA 300
   TTCTAACATA AGGAATACCT TTTTCGCTGA AATTGGCATT GGAGTCTCAG CCAATAGCCT 360
   CCTACTTCTC TTCAACATCT TCAAGTTAAT TTGTGGGCAG AGGTCCAGAC TCACTGACCT 420
   GCCCATTGGT CTCTTGTCCC TAATCAACTT ACTTATGCTA CTGATGACGG CATTCATAGC 480
   CACAGACACT TTTATTTCTT GGAGAGGGTG GGATGACATC ATATGTAAAT CCCTTCTCTA 540
   CCTGTACAGA ACTTTTAGAG GTCTCTCTCT TTGTACCAGC TGCCTGTTGA GTGTCCTGCA 600
   GGCCATCATC CTCAGTCCCA GAAGCTCCTG TTTAGCAAAG TTCAAACATA AGCCTTCCCA 660
   TCACATCTCC TGTGCCATTC TTTCTCTGAG TGTCCTCTAC ATGTTCATTA GCAGTCACCT 720
   CTTAGTATCC ATCATTGCCA CCCCAAATTT GACCACGAAT GACTTTATTC ATGTTACTCA 780
   GTGGTGCTCT ATTCTACCCA TGAGTTACCT CATGCAAAGC ATGTTTTCTA CACTGCTGGC 840
   CATCAGGGAT GTCTTTCTTA TTAGTCTCAT GGTCCTGTCA ACATGGTACA TGGTGGCTCT 900
   CTTGTGTAGG CACAGGAAAC AGACCCGGCA TCTTCAGGGT ACCAGCCTTT CCCCAAAAGC 960
   ATCCCCAGAA CAAAGGGCCA CCCGTTCCAT CCTGATGCTC ATGAGCTTAT TTGTTCTGAT 1020
   GTCTGTCTTT GACAGCATTG TCTGCAGCTC AAGAACTATG TATCTGAATG ATCCAATATC 1080
   TTATTCTTAT CAACTATTTA TGGTGCACAT CTATGCCACA GTAAGCCCTT TTGTGTTTAT 1140
   TGTCACTGAA AAACATATAG TTAACTCTTT GAGGTCCATG TGTGTGAAGG TGCATGAATG 1200
   TTTGAATATT CCTTGATAGC AAGCTCCATT AAGAGGAGCC AATGTAAGCA TCAGAACTGT 1260
   CAATCATGGC GTGCTATGTG CTTTGGCATA TGTGAAATAT GAAGTTGTTT TTCTGTTAAA 1320
   ATGATTTACT TTAACTGACG AGATGATGAA CGTAACAGAA GATTAAACCA CATCCCCTTT 1380
   GATAT 1385
(2) INFORMATION FOR SEQ ID NO:3:
   (i) SEQUENCE CHARACTERISTICS:
      (A) LENGTH: 1331 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:3:
   GTGGATCCCC CGGGCTGCAG GAATTCGGCA CGAGCCGTGA TTAAGGGACT TTGAACTTTT 60
   CAAGGGATTT GGAGTTTTAT GAAGAATTTG AAGATTTACA GAGTTTACAG GAATGGAGCT 120
   GACCAGCCAC TATGACATGC CTTATATCTC CAAGAGCATA AATATAAGGC ATGGCATGAG 180
   AGGACCAGCA GCCACTGTTC TCATATATGA TGAATAAGAA CAGCAGAGTC CACACTGATT 240
   CTACCATAAG GAATACCTTC TCCACTGAAA TTGGCATTGG AATCTTAGCC AACAGTTTCC 300
   TACTTCTCTT CCACATCTTC AAGTTTATTC GTGGACAGAG GTCCAGACTC ACTGACCTGC 360
   CCATTGGTCT CTTGTCCCTA ATCCACCTAC TGATGCTACT GATGGGGGCA TTCATAGCCA   420
   TAGACATTTT TATTTCTTGG AGGGGATGGG ATGACATCAT ATGTAAATTC CTTGTCTACT   480
   TGTACAGAAG TTTTAGAGGT CTCTCTCTTT GTACCACCTG CATGTTGAGT GTCCTGCAGG   540
   CCATCACCCT CAGCCCCAGA AGCTCCTGTT TAGCAAAGTT CAAACATAAG TCTCCCCATC   600
   ACGTCTCCTG TGCCATTATT TCGCTGAGCA TCCTCTACAT GTTCATTAGC AGTCACCTCT   660
   TAGTATCCAT CAATGCCACC CCCAATTTGA CCACGAACAA CTTTATGCAA GTTACTCAGT   720
   CCTGCTACAT TATACCCTTG AGTTACCTCA TGCAAAGCAT GTTTTCTACA CTTCTGGCCA   780
   TCAGAGATAT CTCTCTTATT AGTCTCATGG TCCTCTCGAC TTGTTACATG GAGGTTCTCT   840
   TGTGTAGGCA CAGGAATCAG ATCCAGCATC TTCAAGGGAC CAACCTTTCC CCAAAAGCAT   900
   CTCCAGAACA AAGGGCCACA CAGACCATCC TGATGCTCAT GACCTTCTTT GTCCTAATGT   960
   CCATTTTCGA CAGCATTGTC TCCTGTTCAA GAACTATGTA TCTGAATGAT CCAACATCTT  1020
   ACTATATTCA AATATTTGTA GTGGACATCT ATGCCACAGT CAGCCCTTTT GTGTTTATGA  1080
   GCACTGGAAA ACATATAGTT AACTTTTTGA AGTCCATGTG TGTGAGGGTG AAGAATGTTT  1140
   GAATATTCAT TAATGGACAA GATCCTTTAA GAGGAGCCAA TGTAGTCATC AGAACTGTCA  1200
   GTCATGGTGT GCTGTCTATG TGCTTTGGTA AATGTGAATC ATGAAGTTGT TTTTCTGGTA  1260
   AAATGATTTA CTTTAACCAA CTCATGATTG TAAACATGTA ACAGGAGATT AAACAATATC  1320
   CCCTTCGGAA A                                                       1331
(2) INFORMATION FOR SEQ ID NO:4:
   (i) SEQUENCE CHARACTERISTICS:
      (A) LENGTH: 1496 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:4:
   AATTCGGCAC GAGCAAAGGC AGGGAAGATG CTCCACTGGG ATGTCATGTC TCTATGCTCC    60
   ACAGTGGAAA AGTTGTCACA TTGTACAAAC ACTAAAATTA CGAATTGCTC ACAGGCACTA   120
   AAAGCTTCCT TAATCCTGTG CAGGATCTCC TCAGGTACAG AGTCCTCCTG ATACGTCTAT   180
   CTGGTCAGAG GAAAGAGCTG ATCAGTCATT AACAGAGCTG ATTTGGTCCC TCCAAGGTCA   240
   CATGACAAGG ACTGTATGAG AAAACCAGCA GTGACATGTC TATAGAGATC ATTCTGTGCC   300
   ACACCCAGCT CCATGTTTGG TTTGTGGTAT TTGCTTCCTA TCCACATACA ATGAATAAAG   360
   ACAACACACT CCATGTTGAC ACAATCATGA AAATCACTAT GTTCTCTGAA GTGAGTGTTG   420
   GCATCTTAGC TAACAGTATC CTGTTTTTTG GTCACCTGTG CATGCTCCTT GGAGAGAACA   480
   AGCCTAAGCC CATTCATCTC TACATTGCAT CCTTGTCCCT AACACAACTA ATGCTGCTTA   540
   TAACTATGGG ACTCATAGCT GCTGACATGT TTATTTCTCA GGGGATATGG GATTCTACCT   600
   CATGCCAGTC CCTTATCTAT TTGCACAGGC TTTCGAGGGG TTTTACCCTT AGTGCTGCCT   660
   GTCTGCTGAA TGTCTTTTGG ATGATCACTC TCAGTTCTAA AAAATCCTGT TTAACAAAGT   720
   TTAAACATAA CTCTCCCCAT CACATCTCAG GTGCCTTTCT TCTCCTCTGT GTTCTCTACA   780
   TGTGTTTTAG CAGTCACCTT ATTTTATCGA TTATTGCTAC CCCTAACTTG ACCTCAGATA   840
   ATTTTATGTA TGTTACTAAG TCCTGTTCAT TTCTACCCAT GTGTTACTCC AGAACAAGCA   900
   TGTTTTCCAC AACAATTGCT GTCAGGGAAG CCTTTTTTAT CGGTCTCATG GCCCTGTCCA   960
   GTGGGTACCT GGTGGCTTTC CTCTGGAGAC ACAGGAAGCA GGCCCAGCAT CTTCACAGCA  1020
   CCGGCCTTTC TTCAAAGTCA TCTCCAGAGC AAAGGGCCAC CGAGACCATC CTGCTGCTTA 1080
   TGAGTTTCTT TGTGGTTCTC TACATTTTGG AAAATGTTGT CTTCTACTCA AGGATGAAGT 1140
   TCAAGGATGG GTCAACATTC TACTGTGTCC AAATTATTGT GTCCCATAGC TATGCCACTG 1200
   TCAGCTCTTT TGTGTTTATT TTCACTGAAA AGCGTATGAC TAAGATATTG AGGTCAGTGT 1260
   GTGCCAGAAT AATAAATAAT TGATTATTCA GTGATGGGTA TTGCCCCTTA GAATAAACCA 1320
   TTACGTTGTC ATCAGAGGTT TGGGTCATGA CATAATTGGG ACATTCTCTG TCTTAAATTG 1380
   ATAAATGAAA TTTTCTTTTT TCCTGTTAAA ACTGTTTCCT TTGTGTGTGG ATGCCCAATA 1440
   TATGAAAGAA AACTAAACAC CATGTCCTCT TACATATCCA ACCAAAAAAA AAAAAA 1496
(2) INFORMATION FOR SEQ ID NO:5:
   (i) SEQUENCE CHARACTERISTICS:
      (A) LENGTH: 1053 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:5:
   TTTTTTCCCA CCTCTTCATG CTCTTTGAAA AGAACAGATC TAAGCCCATT GATCTCTACA 60
   TTGCTTTCTT ATCCTTAACC CAACTAATGC TGCTTATAAC TATTGGACTT ATAGCTGCAG 120
   ACATGTTTAT GTCTCGGGGG AGATGGGATT CTACCACATG CCAGTCCCTT ATCTATTTGG 180
   ACAGGCTTTT GAGGGGTTTT ACCCTTTGTG CTACCTGTCT GCTGAATGTC CTTTGGACCA 240
   TCACTCTCAG TCCTAGAAGC TCCTGTTTAA CAACATTTAA ACATAAATCT CCCCATCACA 300
   TCTCAGGTGC CTTTCTTTTC TTCTGTGTTC TCTATATATC TTTTGGCAGT CACCTCTTTT 360
   TATCAACAAT TGCTACCCCC AATTTGACTT CAGATAATTT TATGTATGTT ACTAAATCCT 420
   GTTCATTTCT ACCCATGAGT TACTCCAGAA CAAGCATGTT TTCCACACCA ATGGCCATCA 480
   GGGAAGCCCT TCTTATTGGT CTCATTGGCC TGTCCAGTGG GTACATGGTT GCTTTCCTAT 540
   GGAGACACAA GAATCAGGCC CGGCATCTTC ACAGCACCAG CCTTTCTTCA AAAGTGTCCC 600
   CAGAGCAAAG GGCCACCAGG ACCATCATGA TTCTCATGAG CTTCTTTGTG GTTCTCTACA 660
   TTTTGGAAAA TGTTGTCTTC TACTCTAGGA TGACATTCAA GGATGGGTCA ATGTTCTACT 720
   GTGTCCAAAT TATTGTGTCC CATAGCTATG CCACCATCAG CCCTTTTGTG TTTATTTGCA 780
   CAGAAAAGCG TATAATTAAA CTTTGGGGGT CAATGTCTAG CAGAATAGTA AGTATTTGAT 840
   TACTCAGTGA TGGATATGGT CCCTTAATAT AAACCAATAT GTTGTCATAA TAACTATGGA 900
   TCATGACATA TTGGGGACAT TCTGTGTCTT AAATTTATAA AAAAAATTTT CTTTTTTTGT 960
   GTTTAATCTG TTTCCCTTGT GTGTGGATGA TAAGTATATA AAGGGAAATT AAACAGCGTG 1020
   TCCCCTCAGA TATCCAAAAA AAAAAAAAAA AAA 1053
(2) INFORMATION FOR SEQ ID NO:6:
   (i) SEQUENCE CHARACTERISTICS:
      (A) LENGTH: 3076 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:6:
(2) INFORMATION FOR SEQ ID NO:7:
   (i) SEQUENCE CHARACTERISTICS:
      (A) LENGTH: 1264 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:7:
   TTGGGGTAAA ACGGCTCGAT GACTTCCACA TGTTTTGCCA TGGCAGAATC TGCTCCATGC 60
   GGGACAAGAA AATCTCTTTT CTGGTCTGAC GGGCTTACTG CTGAATTCAC TGTCGGCGAA 120
   GGTAAGTTGA TGACTCATGA TGAACCCTGT TCTATGGCTC CAGATGACAA ACATGATCTC 180
   ATATCAGGGA CTTGTTCGCA CCTTCCCTAA CAGTATCCTG TTTTTTGCCC ACCTCTGCAT 240
   GTTCTTTGAA GAGAACAGGT CTAAGCCCAT TGATCTGTGC ATTGCTTTCT TATCCTTAAC 300
   CCAACTAATG CTGCTTGTAA CTATGGGACT CATAGCTGCA GACATGTTTA TGGCTCAGGG 360
   GATATGGGAT ATTACCACAT GCAGGTCCCT TATCTATTTT CACAGACTTT TGAGGGGTTT 420
   CAACCTTTGT GCTGCCTGTC TACTGCATAT CCTTTGGACC TTCACTCTCA GTCCTAGAAG 480
   CTCCTGTTTA ACAAAGTTTA AACATAAATC TCCCCATCAC ATCTCAGGTG CCTATCTTTT 540
   CTTCTGTGTT CTCTATATGT CCTTTAGCAG TCACCTCTTT GTATTGGTCA TTGCTACCTC 600
   CAATTTAACC TCAGATCATT TTATGTATGT TACTCAGTCC TGCTCACTTC TACCCATGAG 660
   TTACTCCAGA ACAAGCACGT TTTCCTTACT GATGGTCACC AGGGAAGTCT TTCTTATCAG 720
   TCTCATGGCC CTGTCCAGTG GGTACATGGT GACTCTCCTA TGGAGGCACA AGAAGCAGGC 780
   CCAGCATCTT CACAGCACCA GACTTTCTTC AAAAGCATCC CCACAGCAAA GGGCCACCAG 840
   GACCATCCTG CTGCTTATGA CCTTCTTTGT GGTTTTCTAC ATTTTAGGCA CTGTTATCTT 900
   CCACTCAAGG ACTAAGTTCA AGGATGGGTC AATCTTCTAC TGTGTCCAAA TTATTGTGTC 960
   CCATAGCTAT GCCACTATCA GCCCATTTGT GTTTGTTTTT TCTGAAAAGC GCATAATCAA 1020
   GTTTTTTAGA TCAATGTGTG GCAGAATAGT AAATACTTGA TTATTCACTG ATGAGTATGG 1080
   GTCATGAATA TAGTCTAGTA AATTGTGATC AGAGTTATGG CTCATGACAT ATTAAAAACA 1140
   TTCTCTAATT TAAGTTTAAC ATATAAAATT ATCTTATTTC TCTTAAATGT GTTTACTTTG 1200
   TGTGTATTAA AAGTATGTAA AAGATAATTA ATCCCCAAAT ACACCTTTTT TTCAAATTAA 1260
   AAAA 1264
(2) INFORMATION FOR SEQ ID NO:B:
   (i) SEQUENCE CHARACTERISTICS:
      (A) LENGTH: 315 amino acids
      (B) TYPE: amino acid
      (C) STRANDEDNESS: single
      (D) TOPOLOGY: linear
   (ii) MOLECULE TYPE: protein
   (xi) SEQUENCE DESCRIPTION: SEQ ID NO:8:
(2) INFORMATION FOR SEQ ID NO:9:
   (i) SEQUENCE CHARACTERISTICS:
      (A) LENGTH: 311 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:9:
   Met Met Asn Lys Asn Ser Arg Leu His Ile Asp Ser Asn Ile Arg Asn
   1 5 10 15
   Thr Phe Phe Thr Glu Ile Gly Ile Gly Val Ser Ala Asn Ser Leu Leu
   20 25 30
   Leu Leu Phe Asn Ile Phe Lys Phe Ile His Gly Gln Arg Ser Arg Leu
   35 40 45
   Thr Asp Leu Pro Ile Gly Leu Leu Ser Leu Ile Asn Leu Leu Met Leu
   50 55 60
   Leu Ile Met Ala Cys Ile Ala Thr Asp Ile Phe Ile Ser Cys Arg Arg
   65 70 75 80
   Trp Asp Asp Ile Ile Cys Lys Ser Leu Leu Tyr Leu Tyr Arg Thr Phe
   85 90 95
   Arg Gly Leu Ser Leu Ser Thr Thr Cys Leu Leu Ser Val Leu Gln Ala
   100 105 110
   Ile Ile Leu Ser Pro Arg Ser Ser Cys Leu Ala Lys Tyr Lys His Lys
   115 120 125
   Pro Pro His His Ile Phe Cys Ala Met Leu Phe Leu Ser Val Leu Tyr
   130 135 140
   Met Phe Ile Ser Ser His Leu Leu Leu Ser Ile Ile Ala Thr Pro Asn
   145 150 155 160
   Leu Thr Thr Asn Asp Phe Ile His Val Ser Gln Ser Cys Ser Ile Leu
   165 170 175
   Pro Met Ser Tyr Leu Met Gln Ser Met Phe Ser Thr Leu Leu Ala Ile
   180 185 190
   Arg Asn Val Phe Leu Ile Ser Leu Ile Val Leu Ser Thr Trp Tyr Met
   195 200 205
   Val Ala Leu Leu Cys Arg His Arg Lys Gln Thr Arg His Leu Gln Asp
   210 215 220
   Thr Ser Leu Ser Arg Lys Ala Ser Pro Glu Gln Arg Ala Thr Arg Ser
   225 230 235 240
   Ile Leu Met Leu Arg Ser Leu Phe Gly Leu Met Ser Ile Phe Asp Ser
   245 250 255
   Ile Ala Ser Cys Ser Arg Thr Met Tyr Leu Asn Asp Pro Thr Ser Tyr
               260                 265                 270
   Ser Ile Gln Leu Leu Val Val His Ile Tyr Ala Thr Val Ser Pro Phe
           275                 280                 285
   Val Phe Met Ile Thr Glu Lys His Ile Val Asn Tyr Leu Lys Ser Met
       290                 295                 300
   Tyr Val Arg Val Leu Asn Val
   305                 310
(2) INFORMATION FOR SEQ ID NO:10:
   (i) SEQUENCE CHARACTERISTICS:
      (A) LENGTH: 311 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:10:
   Met Met Asn Lys Asn Ser Arg Val His Thr Asp Ser Thr Ile Arg Asn
   1               5                   10                  15
   Thr Phe Ser Thr Glu Ile Gly Ile Gly Ile Leu Ala Asn Ser Phe Leu
               20                  25                  30
   Leu Leu Phe His Ile Phe Lys Phe Ile Arg Gly Gln Arg Ser Asp Leu
           35                  40                  45
   Thr Asp Leu Pro Ile Gly Leu Leu Ser Leu Ile His Leu Leu Met Leu
       50                  55                  60
   Leu Met Gly Ala Phe Ile Ala Ile Asp Ile Phe Ile Ser Trp Arg Gly
   65                  70                  75                  80
   Trp Asp Asp Ile Ile Cys Lys Phe Leu Val Tyr Leu Tyr Arg Ser Phe
   85 90 95
   Arg Gly Leu Ser Leu Cys Thr Thr Cys Met Leu Ser Val Leu Gln Ala
   100 105 110
   Ile Thr Leu Ser Pro Arg Ser Ser Cys Leu Ala Lys Phe Lys His Lys
   115 120 125
   Ser Pro His His Val Ser Cys Ala Ile Ile Ser Leu Ser Ile Leu Tyr
   130 135 140
   Met Phe Ile Ser Ser His Leu Leu Val Ser Ile Asn Ala Thr Pro Asn
   145 150 155 160
   Leu Thr Thr Asn Asn Phe Met Gln Val Thr Gln Ser Cys Tyr Ile Ile
   165 170 175
   Pro Leu Ser Tyr Leu Met Gln Ser Met Phe Ser Thr Leu Leu Ala Ile
   180 185 190
   Arg Asp Ile Ser Leu Ile Ser Leu Met Val Leu Ser Thr Cys Tyr Met
   195 200 205
   Glu Val Leu Leu Cys Arg His Arg Asn Gln Ile Gln His Leu Gln Gly
   210 215 220
   Thr Asn Leu Ser Pro Lys Ala Ser Pro Glu Gln Arg Ala Thr Gln Thr
   225 230 235 240
   Ile Leu Met Leu Met Thr Phe Phe Val Leu Met Ser Ile Phe Asp Ser
   245 250 255
   Ile Val Ser Cys Ser Arg Thr Met Tyr Leu Asn Asp Pro Thr Ser Tyr
   260 265 270
   Tyr Ile Gln Ile Phe Gly Val Asp Ile Tyr Ala Thr Val Ser Pro Phe
   275 280 285
   Val Phe Met Ser Thr Glu Lys His Ile Val Asn Phe Leu Lys Ser Met
   290 295 300
   Cys Val Arg Val Lys Asn Val
   305 310
(2) INFORMATION FOR SEQ ID NO:11:
   (i) SEQUENCE CHARACTERISTICS:
      (A) LENGTH: 310 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:11:
   Met Asn Lys Asp Asn Thr Leu His Val Asp Thr Ile Met Lys Ile Thr
   1               5                   10                  15
   Met Phe Ser Glu Val Ser Val Gly Ile Leu Ala Asn Ser Ile Leu Phe
               20                  25                  30
   Phe Gly His Leu Cys Met Leu Leu Gly Glu Asn Lys Pro Lys Pro Ile
           35                  40                  45
   His Leu Tyr Ile Ala Ser Leu Ser Leu Thr Gln Leu Met Leu Leu Ile
       50                  55                  60
   Thr Met Gly Leu Ile Ala Ala Asp Met Phe Ile Ser Gln Gly Ile Trp
   65                  70                  75                  80
   Asp Ser Thr Ser Cys Gln Ser Leu Ile Tyr Leu His Arg Leu Ser Arg
                   85                  90                  95
   Gly Phe Thr Leu Ser Ala Ala Cys Leu Leu Asn Val Phe Trp Met Ile
               100                 105                 110
   Thr Leu Ser Ser Lys Lys Ser Cys Leu Thr Lys Phe Lys His Asn Ser
           115                 120                 125
   Pro His His Ile Ser Gly Ala Phe Leu Leu Leu Cys Val Leu Tyr Met
       130                 135                 140
   Cys Phe Ser Ser His Leu Ile Leu Ser Ile Ile Ala Thr Pro Asn Leu
   145                 150                 155                 160
   Thr Ser Asp Asn Phe Met Tyr Val Thr Lys Ser Cys Ser Phe Leu Pro
                   165                 170                 175
   Met Cys Tyr Ser Arg Thr Ser Met Phe Ser Thr Thr Ile Ala Val Arg
               180                 185                 190
   Glu Ala Phe Phe Ile Gly Leu Met Ala Leu Ser Ser Gly Tyr Leu Val
           195                 200                 205
   Ala Phe Leu Trp Arg His Arg Lys Gln Ala Gln His Leu His Ser Thr
       210                 215                 220
   Gly Leu Ser Ser Lys Ser Ser Pro Glu Gln Arg Ala Thr Glu Thr Ile
   225                 230                 235                 240
   Leu Leu Leu Met Ser Phe Phe Val Val Leu Tyr Ile Leu Glu Asn Val
                   245                 250                 255
   Val Phe Tyr Ser Ser Arg Met Phe Lys Asp Gly Ser Thr Phe Tyr Cys
               260                 265                 270
   Val Gln Ile Ile Val Ser His Ser Tyr Ala Thr Val Ser Ser Phe Val
           275                 280                 285
   Phe Ile Phe Thr Glu Lys Arg Met Thr Lys Ile Leu Arg Ser Val Cys
       290                 295                 300
   Ala Arg Ile Ile Asn Asn
   305                 310
(2) INFORMATION FOR SEQ ID NO:12:
   (i) SEQUENCE CHARACTERISTICS:
      (A) LENGTH: 278 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:12:
   Phe Ser His Leu Phe Met Leu Phe Glu Lys Asn Arg Ser Lys Pro Ile
   1               5                   10                  15
   Asp Leu Tyr Ile Ala Phe Leu Ser Leu Thr Gln Leu Met Leu Leu Ile
               20                  25                  30
   Thr Ile Gly Leu Ile Ala Ala Asp Met Phe Met Ser Arg Gly Arg Trp
           35                  40                  45
   Asp Ser Thr Thr Cys Gln Ser Leu Ile Tyr Leu Asp Arg Leu Leu Arg
       50                  55                  60
   Gly Phe Thr Leu Cys Ala Thr Cys Leu Leu Asn Val Leu Trp Thr Ile
   65                  70                  75                  80
   Thr Leu Ser Pro Arg Ser Ser Cys Leu Thr Thr Phe Lys His Lys Ser
                   85                  90                  95
   Pro His His Ile Ser Gly Ala Phe Leu Phe Phe Cys Val Leu Tyr Ile
               100                 105                 110
   Ser Phe Gly Ser His Leu Phe Leu Ser Thr Ile Ala Thr Pro Asn Leu
           115                 120                 125
   Thr Ser Asp Asn Phe Met Tyr Val Thr Lys Ser Cys Ser Phe Leu Pro
       130                 135                 140
   Met Ser Tyr Ser Arg Thr Ser Met Phe Ser Thr Pro Met Ala Ile Arg
   145                 150                 155                 160
   Glu Ala Leu Leu Ile Gly Leu Ile Gly Leu Ser Ser Gly Tyr Met Val
                   165                 170                 175
   Ala Phe Leu Trp Arg His Lys Asn Gln Ala Arg His Leu His Ser Thr
               180                 185                 190
   Ser Leu Ser Ser Lys Val Ser Pro Glu Gln Arg Ala Thr Arg Thr Ile
           195                 200                 205
   Met Ile Leu Met Ser Phe Phe Val Val Leu Tyr Ile Leu Glu Asn Val
       210                 215                 220
   Val Phe Tyr Ser Arg Met Thr Phe Lys Asp Gly Ser Met Phe Tyr Cys
   225                 230                 235                 240
   Val Gln Ile Ile Val Ser His Ser Tyr Ala Thr Ile Ser Pro Phe Val
                   245                 250                 255
   Phe Ile Cys Thr Glu Lys Arg Ile Ile Lys Leu Trp Gly Ser Met Ser
               260                 265                 270
   Ser Arg Ile Val Ser Ile
           275
(2) INFORMATION FOR SEQ ID NO:13:
   (i) SEQUENCE CHARACTERISTICS:
      (A) LENGTH: 310 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:13:
   Met Arg Arg Ile Ser Thr Leu Tyr Gly Val Val Asp Lys Gln Ala Ile
   1               5                   10                  15
   Phe Phe Ser Glu Val Val Ile Gly Ile Ser Phe Asn Ser Ile Leu Phe
   20 25 30
   Leu Phe His Ile Phe Gln Phe Leu Leu Glu Arg Arg Leu Arg Ile Thr
   35 40 45
   Asp Leu Ile Ile Ser Leu Leu Ala Leu Ile His Leu Gly Met Leu Thr
   50 55 60
   Val Met Gly Phe Arg Ala Val Asp Ile Phe Ala Ser Gln Asn Val Trp
   65 70 75 80
   Asn Asp Ile Lys Cys Lys Ser Leu Ala His Leu His Arg Leu Leu Arg
   85 90 95
   Gly Leu Ser Leu Cys Ala Thr Cys Leu Leu Ser Ile Phe Gln Ala Ile
   100 105 110
   Thr Leu Ser Pro Arg Ser Ser Cys Leu Ala Lys Phe Lys Tyr Lys Ser
   115 120 125
   Thr Gln His Ser Leu Cys Ser Leu Leu Val Leu Trp Ala Phe Tyr Met
   130 135 140
   Ser Cys Gly Thr His Tyr Ser Phe Thr Ile Val Ala Asp Tyr Asn Phe
   145 150 155 160
   Ser Ser Arg Ser Leu Ile Phe Val Thr Glu Ser Cys Ile Ile Leu Pro
   165 170 175
   Met Asp Tyr Ile Thr Arg His Leu Phe Phe Ile Leu Gly Ile Phe Arg
   180 185 190
   Asp Val Ser Phe Ile Gly Leu Met Ala Leu Ser Ser Gly Tyr Met Val
   195 200 205
   Ala Leu Leu Cys Arg His Arg Lys Gln Ala Gln His Leu His Arg Thr
   210 215 220
   Ser Leu Ser Pro Lys Ala Ser Pro Glu Gln Arg Ala Thr Arg Thr Ile
   225 230 235 240
   Leu Leu Leu Met Ser Phe Phe Val Leu Met Tyr Cys Leu Asp Cys Thr
   245 250 255
   Ile Ser Ala Ser Arg Leu Met His Asn Gly Glu Pro Ile His His Ser
   260 265 270
   Ile Gln Met Met Val Ser Asn Ser Tyr Ala Thr Leu Ser Pro Leu Leu
   275 280 285
   Leu Ile Val Thr Glu Asn Arg Ile Ser Arg Phe Leu Lys Ser Leu Leu
   290 295 300
   Gly Arg Thr Val Asp Ala
   305 310
(2) INFORMATION FOR SEQ ID NO:14:
   (i) SEQUENCE CHARACTERISTICS:
      (A) LENGTH: 307 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:14:
   Met Met Asn Pro Val Leu Trp Leu Gln Met Thr Asn Met Ile Ser Tyr
   1 5 10 15
   Gln Gly Leu Val Arg Thr Phe Pro Asn Ser Ile Leu Phe Phe Ala His
   20 25 30
   Leu Cys Met Phe Phe Glu Glu Asn Arg Ser Lys Pro Ile Asp Leu Cys
   35 40 45
   Ile Ala Phe Leu Ser Leu Thr Gln Leu Met Leu Leu Val Thr Met Gly
   50 55 60
   Leu Ile Ala Ala Asp Met Phe Met Ala Gln Gly Ile Trp Asp Ile Thr
   65 70 75 80
   Thr Cys Arg Ser Leu Ile Tyr Phe His Arg Leu Leu Arg Gly Phe Asn
   85 90 95
   Leu Cys Ala Ala Cys Leu Leu His Ile Leu Trp Thr Phe Thr Leu Ser
   100 105 110
   Pro Arg Ser Ser Cys Leu Thr Lys Phe Lys His Lys Ser Pro His His
   115 120 125
   Ile Ser Gly Ala Tyr Leu Phe Phe Cys Val Leu Tyr Met Ser Phe Ser
   130 135 140
   Ser His Leu Phe Val Leu Val Ile Ala Thr Ser Asn Leu Thr Ser Asp
   145 150 155 160
   His Phe Met Tyr Val Thr Gln Ser Cys Ser Leu Leu Pro Met Ser Tyr
   165 170 175
   Ser Arg Thr Ser Thr Phe Ser Leu Leu Met Val Thr Arg Glu Val Phe
   180 185 190
   Leu Ile Ser Leu Met Ala Leu Ser Ser Gly Tyr Met Val Thr Leu Leu
   195 200 205
   Trp Arg His Lys Lys Gln Ala Gln His Leu His Ser Thr Arg Leu Ser
   210 215 220
   Ser Lys Ala Ser Pro Gln Gln Arg Ala Thr Arg Thr Ile Leu Leu Leu
   225 230 235 240
   Met Thr Phe Phe Val Val Phe Tyr Ile Leu Gly Thr Val Ile Phe His
   245 250 255
   Ser Arg Thr Lys Phe Lys Asp Gly Ser Ile Phe Tyr Cys Val Gln Ile
   260 265 270
   Ile Val Ser His Ser Tyr Ala Thr Ile Ser Pro Phe Val Phe Val Phe
   275 280 285
   Ser Glu Lys Arg Ile Ile Lys Phe Phe Arg Ser Met Cys Gly Arg Ile
   290 295 300
   Val Asn Thr
   305
(2) INFORMATION FOR SEQ ID NO:15:
   (i) SEQUENCE CHARACTERISTICS:
      (A) LENGTH: 173 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:15:
   Asn Ile Ser Pro Val Ile Tyr Arg Tyr Arg Leu Met Arg Gly Leu Ser
   1 5 10 15
   Ile Ser Thr Thr Cys Leu Leu Ser Val Leu Gln Ala Ile Asn Leu Thr
   20 25 30
   Pro Arg Ser Ser Arg Leu Ala Arg Ser Ser His His Lys Pro Arg Cys
   35 40 45
   Phe Leu Leu Leu Trp Val Phe His Ile Ser Ile Ser Gly Ser Phe Leu
   50 55 60
   Val Ser Thr Leu Pro Ser Lys Asn Val Ala Ser Asn Ser Val Thr Phe
   65 70 75 80
   Val Thr Gln Ser Cys Ser Ala Gly Pro Leu Ser Cys Phe Leu Gly Gln
   85 90 95
   Thr Ile Phe Thr Leu Met Thr Phe Gln Asp Val Ser Leu Gln Leu Met
   100 105 110
   Ala Pro Phe Ser Gly Tyr Met Val Ile Leu Leu Cys Arg His Asn Arg
           115                 120                 125
   Gln Ser Gln His Leu His Ser Ile Asn Leu Ser Pro Lys Ala Pro Pro
       130                 135                 140
   Asp Lys Arg Ala Ile Gln Ser Ile Leu Leu Leu Val Ser Phe Phe Val
   145                 150                 155                 160
   Phe Met Cys Leu Phe Pro Phe Ala Ala Leu Thr Leu Leu
                   165                 170
(2) INFORMATION FOR SEQ ID NO:16:
   (i) SEQUENCE CHARACTERISTICS:
      (A) LENGTH: 71 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:16:
   Ser Lys Arg Lys Lys Ser Phe Leu Leu Cys Ile Gly Trp Leu Ala Leu
   1               5                   10                  15
   Thr Asp Leu Val Gly Gln Leu Leu Thr Ser Pro Val Val Ile Leu Val
               20                  25                  30
   Tyr Leu Ser Gln Arg Arg Trp Glu Gln Leu Asp Pro Ser Gly Arg Leu
           35                  40                  45
   Cys Thr Phe Phe Gly Leu Thr Met Thr Val Phe Gly Leu Ser Ser Leu
       50                  55                  60
   Leu Val Ala Ser Ala Met Ala
   65                  70
(2) INFORMATION FOR SEQ ID NO:17:
   (i) SEQUENCE CHARACTERISTICS:
      (A) LENGTH: 74 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:17:
   Gly Gln Arg Ser Arg Leu Thr Asp Leu Pro Ile Gly Leu Leu Ser Leu
   1               5                   10                  15
   Ile Asn Leu Leu Met Leu Leu Ile Met Ala Cys Ile Ala Thr Asp Ile
               20                  25                  30
   Phe Ile Ser Cys Arg Arg Trp Asp Asp Ile Ile Cys Lys Ser Leu Leu
           35                  40                  45
   Tyr Leu Tyr Arg Thr Phe Arg Gly Leu Ser Leu Ser Thr Thr Cys Leu
       50                  55                  60
   Leu Ser Val Leu Gln Ala Ile Ile Leu Ser
   65                  70
(2) INFORMATION FOR SEQ ID NO:18:
   (i) SEQUENCE CHARACTERISTICS:
      (A) LENGTH: 174 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:18:
   Lys Cys Lys Ser Leu Ala His Leu His Arg Leu Leu Arg Gly Leu Ser
   1               5                   10                  15
   Leu Cys Ala Thr Cys Leu Leu Ser Ile Phe Gln Ala Ile Thr Leu Ser
               20                  25                  30
   Pro Arg Ser Ser Cys Leu Ala Lys Ser Thr Gln His Ser Leu Cys Ser
           35                  40                  45
   Leu Leu Val Leu Trp Ala Phe Tyr Met Ser Cys Gly Thr His Tyr Ser
       50                  55                  60
   Phe Thr Ile Val Ala Asp Tyr Asn Phe Ser Ser Arg Ser Leu Ile Phe
   65                  70                  75                  80
   Val Thr Glu Ser Cys Ile Ile Leu Pro Met Asp Tyr Ile Thr Arg Asp
                   85                  90                  95
   Leu Phe Phe Ile Leu Gly Ile Phe Arg Asp Val Ser Phe Ile Gly Leu
               100                 105                 110
   Met Ala Leu Ser Ser Gly Tyr Met Val Ala Leu Leu Cys Arg His Arg
           115                 120                 125
   Lys Gly Ala Gln His Leu His Arg Thr Ser Leu Ser Pro Lys Ala Ser
       130                 135                 140
   Pro Glu Gln Arg Ala Thr Arg Thr Ile Leu Leu Leu Met Ser Phe Phe
   145                 150                 155                 160
   Val Leu Met Tyr Cys Leu Asp Cys Thr Ile Ser Ala Ser Arg
                   165                 170